## Europäisches Patentamt

## European Patent Office

(11) Publication number: **0 207 044**
A1

## Office européen des brevets

(12) **EUROPEAN PATENT APPLICATION**

(21) Application number: **86870086.5**

(22) Date of filing: **16.06.86**

(51) Int. Cl.⁴: **C 12 N 15/00**, C 12 P 21/00, C 07 K 15/00, C 12 N 1/20 // (C12N15/00, C12R1:19)

(30) Priority: **20.06.85 US 747135**
**02.05.86 US 856385**

(43) Date of publication of application: **30.12.86**
**Bulletin 86/52**

(84) Designated Contracting States: **AT BE CH DE FR GB IT LI LU NL SE**

(71) Applicant: **Monsanto Company, Patent Department 800 North Lindbergh Boulevard, St. Louis Missouri 63167 (US)**

(72) Inventor: **Mai, Marilyn Sue, 1505 Morning Sun Drive, Baliwin Missouri 63011 (US)**
Inventor: **Bittner, Michael Louis, 12459 Glenbush, Maryland Heights Missouri 63043 (US)**
Inventor: **Braford, Sarah Ruth, 812 N. Mosley Road, Creve Coeur Missouri 63141 (US)**

(74) Representative: **Lunt, John Cooper et al, Monsanto Europe S.A. Patent Department Avenue de Tervuren 270-272 Letter Box No 1, B-1150 Brusseis (BE)**

(54) **Release of peptides from polypeptides.**

(57) A method for obtaining heterologous peptides from fusion proteins wherein heterologous peptides include eucaryotic hormones such as atrial peptides. A novel DNA sequence encoding atrial peptide III. Various genes, DNA vectors, endopeptidases and transformed bacteria useful in practicing the method of the present invention.

EP 0 207 044 A1

## Release of Peptides From Polypeptides

### Technical Field

The present invention relates to methods and compositions for obtaining heterologous peptides from fusion proteins. In one important embodiment, the present invention relates to the cleavage of bacterially-produced fusion proteins containing an amino acid sequence specific for Staphylococcus aureus V8 protease cleavage at the junction of two peptides which together comprise the bacterially produced fusion protein. In another important embodiment this invention relates to production of atrial peptides as a bacterially produced fusion protein and the subsequent isolation of said atrial peptides employing the cleavage methods of the present invention. In yet another embodiment, this invention relates to the creation of novel DNA coding sequences for atrial peptides.

### Background of the Invention

Advances in recombinant DNA technology and genetic engineering have provided a means for producing in bacteria eucaryotic proteins of clinical and hence economic importance. The employment of bacterial cells as factories (e.g. host cells) for eucaryotic protein production has become especially attractive for eucaryotic proteins of limited availability. One important example of limited availability is human hormones. The problem of obtaining tissue is further magnified by the fact that a given tissue extract yields very low quantities of a given hormone.

The use of bacteria as host cells for eucaryotic protein production currently involves first isolating or synthesizing the gene or DNA sequence encoding the desired peptide and, second, incorporating the gene or DNA sequence into the genome of the host cell in a manner which allows for expression of the DNA sequence or gene and resultant protein production, accumulation and/or secretion.

Differences between eucaryotic and procaryotic cellular control of gene expression and protein production, however, have given rise to several obstacles which must be overcome if given eucaryotic proteins, peptides or fragments thereof are to be produced in bacteria efficiently and at commercially-attractive levels.

In eucaryotes, many mature proteins are first translated as pre-proteins; i.e., polypeptides comprised of the mature proteins's sequence fused to a leader or signal sequence. Eucaryotic mRNA encodes the entire pre-protein, which is processed after translation to remove the leader sequence and provide the mature protein. Although eucaryotic cells are equipped to specifically process such pre-proteins into mature proteins, bacterial cells are generally not able to recognize the processing signals present in eucaryotic proteins. Thus, if complete complementary DNA (cDNA) transcripts of eucaryotic mRNA are employed as the DNA sequences for expression in bacteria, the pre-protein, not the mature protein, is obtained. It is possible to convert pre-proteins to mature proteins in vitro, but not without significant expense.

In the event that the DNA sequence encoding the mature protein is used for mature protein expression in bacteria, this sequence will be lacking the eucaryotic translation and post-translation processing signals usually contained within the DNA for the leader sequence. Therefore, for expression of cloned eucaryotic genes or other heterologous DNA sequences in bacterial systems, it has proven desirable to employ bacterial control signals for reasons of efficiency and because eucaryotic signals may not be recognized by a bacterial host cell.

The term "heterologous DNA" is defined herein as DNA at least a portion of which is not normally contained within the genome of the host cell. Examples of heterologous DNA include, but are not limited to, viral and eucaryotic genes, gene fragments, alleles and synthetic DNA sequences. The term "heterologous protein" or "heterologous polypeptide" is defined herein as a protein or polypeptide at least a portion of which is not normally encoded within the genome of the host cell. The term "genome" refers to all DNA (chromosomal and extrachromosomal) contained within a specified cell.

The bacterial control signals include a promoter, which signals the initiation of transcription, and translation control signals comprising a ribosome binding site, a translation start signal and a translation stop signal. All of these signals except the translation stop signal must be situated in front of the eucaryotic gene or other DNA to be expressed.

The art has adopted several approaches to expressing heterologous DNA (e.g. eucaryotic genes) in bacteria. In one approach, the translation start signal, ATG, under the control of a bacterial promoter, is located immediately preceding the DNA sequence encoding a heterologous (e.g. eucaryotic) protein. Expression of such a DNA construct results in production of eucaryotic proteins free from endogenous proteins or protein fragments herein defined as "direct" protein production. The proteins so produced, however, typically contain an amino(N)-terminal methionine as the ATG translation start signal is also a methionine codon. See Harris, T.J.R.

(1983). Thus, unless the desired mature protein begins with methionine, the protein will now have an N-terminus altered by inclusion of that methionine residue.

Additionally, the direct production approach has not generally been successfully applied to production of heterologous (e.g. eucaryotic) peptides, wherein a "peptide" is defined as a protein containing fewer than 100 amino acids or proteins having a molecular weight of less than about 10,000 daltons. The problem with direct production of heterologous peptides lies in the tendency of bacteria, such as E. coli, to recognize eucaryotic peptides produced therein as foreign and, thus, proceed to degrade these peptides as soon as these peptides are produced or shortly thereafter. See. R.K. Craig and L. Hall (1983); Itakura et al. (1977). Furthermore, it has been found that structural features inherent in the nucleic acid (DNA or RNA) sequence coding for a heterologous protein or peptide product often interfere with efficient heterologous protein or peptide production (i.e. translation) in bacteria. Hence, an alternate approach to production of such heterologous peptides as eucaryotic peptide hormones has been developed.

In one alternate approach, the DNA segment encoding the desired protein or peptide is ligated to endogenous DNA encoding all or part of a bacterial protein under the control of its bacterial promoter. The endogenous bacterial DNA necessarily also contains the ribosome binding site and translation start signal. In ligation, the DNA encoding the desired protein or peptide must be inserted in-frame with the endogenous transcription and translation control signals and endogenous DNA coding sequences, and in the same orientation. Expression of the ligated DNA provides a fusion protein comprising the heterologous protein or peptide linked (e.g. fused) to a whole or partial bacterial protein. Ideally, such fusion constructs should provide a relatively high and/or stable level of fusion protein accumulation in the bacterial host cell and/or high level of secretion by the host cell.

Production of heterologous proteins and peptides in bacteria has been reported to be aided by fusion of the desired heterologous product peptide or protein to an endogenous protein or fragment thereof. For example, the endogenous protein may serve to enhance transcription and/or translation, Craig and Hall (1983), or, may be employed to aid in purification of desired product. See Sessenfeld, H.M. and Brewer, S.J. (1984) (use of polyarginine binding to ion exchange columns); Germino, J. and Bastia, D. (1984) (β-galactosidase affinity column). In addition, especially in Bacillus and yeast systems, fusion of a desired protein or peptide to an endogenously secreted protein or a signal peptide may result in the secretion of mature protein product into the host growth media free from intracellular proteins and endogenous protein sequences.

Furthermore, the fusion protein approach is useful in protecting otherwise foreign protein or peptide products from intracellular degradation. See Itakura, K. et al. (1977) and R.K. Craig and L. Hall (1983). Fusion proteins engineered for protective purposes can employ endogenous polypeptide sequences at either the amino or carboxy terminus of the heterologous peptide.

In all cases, final isolation of the bacterially-produced eucaryotic peptide must be achieved by site-specific enzymatic or chemical cleavage at the endogenous-eucaryotic peptide fusion site, herein referred to as "junction site", or by selective degradation of the endogenous polypeptide sequences. The junction site may contain a single peptide bond that links the heterologous (e.g. eucaryotic) peptide to the endogenous protein or contain a series of peptide bonds joining the heterologous peptide to the endogenous protein. Most commonly, bacterially produced fusion proteins are constructed so that the endogenous peptide or fragment thereof comprises the N-terminal portion of the fusion protein with the heterologous peptide comprising the C-terminal portion. Such constructions allow for the simultaneous release of the endogenous peptide/protein and the N-terminal methionine following cleavage at the junction site.

Examples of site-specific release of eucaryotic peptides from bacterially produced fusion proteins by chemical means include the following: Stephien et al. (1983) (proinsulin fused to yeast galactokinase); Tanaka et al. (1982) (α-neo-endorphin fused to E. coli β-galactosidase); Goeddel et al. (1979) (insulin A and B chain fused to E. coli β-galactosidase); Itakura et al. (1977) (somatostatin fused to E. coli β-galactosidase). In all the foregoing examples, the chemical cyanogen bromide was employed to cleave the fusion protein and release the desired peptide. Cleavage of a protein or polypeptide is defined herein as the hydrolysis of a peptide bond in a protein or polypeptide. Cyanogen bromide hydrolyzes peptide bonds at the carboxy-side of methionine residues under acid conditions. Thus, site-specific cleavage of a fusion protein requires the presence of a methionine residue immediately upstream and adjacent to the N-terminal amino acid of the desired peptide and an absence of methionine residues in the internal amino acid sequence of the desired peptide.

The disadvantages of chemical hydrolysis include the harsh acid conditions under which cleavage occurs, such conditions possible causing undesirable modifications in the product peptide, the need to know the amino acid sequence of the product peptide to insure against internal cleavage sites, and the observation that the specificity of some chemical cleavages depend largely upon amino acids immediately adjacent to the bond being cleaved.

As an alternative to chemical cleavage, several investigators have reported the use of enzymes, peptidases, to achieve release of the desired peptide product from bacterially produced fusion proteins. Peptidases are generally defined as enzymes which catalyze the hydrolysis (cleavage) of peptide bonds.

One specific class of peptidases employed to date has been the endopeptidases. These peptidases are particularly well suited for use in release of a desired peptide from fusion proteins comprising an endogenous (carrier) protein at the N-terminus of the fusion protein and the desired peptide at the C-terminus. Endopeptidases recognize either specific single amino acids or specific amino acid sequences present within the internal amino acid sequence of a polypeptide and then cleave the peptide bond preferably on the carboxyside of a given amino acid. The amino acid or amino acid sequence specifically recognized and cleaved by a given endopeptidase shall henceforth be referred to as a "trigger amino acid" or "trigger sequence", respectively, or collectively as a "trigger signal."

Examples of various endopeptidases employed to cleave bacterially produced fusion proteins to release a desired peptide include the following: International Patent Application publication number WO84/00380 (published 2/2/84) (trypsin to release human calcitonin from a tryptophan promoter/operator system); European Patent Application publication number 35,384 (published 9/9/81) (suggested use of enterokinase); Nagai and Thogersen (1984) (Factor Xa to release human $\beta$-globin from a $\lambda$CII protein); Germino, J. and Bastia, D. (1984) (microbial collagenase to release R6K replication initiator from $\beta$-galactosidase); Shine et al. (1980) (trypsin to release $\beta$-endorphin from $\beta$-galactosidases); Rutter, W. J. (1979) (suggested use of enterokinase to cleave fusion proteins); European Patent Application publication number 161,937 (published 11/21/85) (Factor Xa to release $\beta$-globin from $\lambda$CII, human calcitonin glycine from CAT, and myosin light chain from $\lambda$CII).

As in the case of chemical cleavage, the trigger signal must constitute the junction bond or site if release of the mature peptide from the bacterially produced fusion protein is to be achieved. Unlike chemical cleavage, however, the vast number of endopeptidases available affords a greater choice of trigger signals for potential use in peptide release.

The decision of which trigger signal or endopeptidase is best employed to achieve release is governed by several factors most of which are tied to the specific bacterial expression system employed to produce the fusion protein and the amino acid sequence of the desired peptide itself. As will be discussed herein, there exists a significant degree of unpredictability in the art. This predictability is best understood by reviewing some of the factors which affect selection of a given endopeptidase and subsequent cleavage of fusion proteins by endopeptidases.

The major factors affecting the choice of the trigger signal and hence endopeptidase employed is whether the complete amino acid sequence of the desired peptide is known and whether the resultant fusion protein allows endopeptidase cleavage at the junction site. If the amino acid sequence is known, a trigger signal can be chosen which does not occur in the desired peptide thereby avoiding unwanted hydrolysis of the desired peptide. Once chosen, a DNA sequence encoding the trigger signal must be synthesized and inserted at the junction site in a manner which will not significantly interfere with expression, production, accumulation and/or purification of the fusion protein. For example, when inserting a given trigger signal at the junction site, the insertion must not disturb the in-frame reading of the coding sequences for the endogenous and heterologous peptides. In preparing the fusion protein for endopeptidase cleavage, it is necessary that the trigger signal be available or exposed for optimum cleavage and release of the desired peptide, and that the cleavage conditions be such that the desired peptide is not irreparably damaged by the reaction conditions necessary for cleavage. Additionally, it is generally desirable that trigger signals are not present within the endogenous protein so that a clean release of the desired peptide can be achieved. The maintenance of the integrity of the endogenous protein is often required for a subsequent commercially feasible purification of the the desired peptide.

In the event that the precise amino acid sequence of the desired peptide is not known, the trigger signal should comprise an amino acid sequence of sufficient complexity so as to diminish the likelihood of a similar sequence being contained within the desired peptide.

As an additional consideration when employing endopeptidases, it has been determined that for some endopeptidases, amino acids in the vicinity of the site of hydrolysis will be recognized and/or bound by the enzyme. These "peripheral" amino acids, in some instances, can increase the catalytic efficiency or binding affinity of the enzyme and thus effect the susceptibility of a peptide containing a trigger signal to hydrolysis by a given endopeptidase. European Patent Application publication number 35,384 (published 9/9/81). Conversely, these peripheral amino acids may decrease the hydrolytic efficiency of a given endopeptidase. Behrens and Brown (1976); Austen and Smith (1976); Houmard and Drapeau (1972a). The presence of peripheral amino acids at or near the junction site should, therefore, be considered as to their effects, if known, on the trigger signal. The effect of peripheral amino acids on specific endopeptidase cleavage is, however, unknown for many of the described endopeptidases. Thus, the ambiguities in the context (e.g. structure) and content (e.g. linear sequence) of any given endopeptidase trigger signal render the operability of a trigger signal for release of a desired peptide from a fusion protein unpredictable in most instances.

In summary, construction of a specific fusion protein with a given trigger signal providing for site-specific release of a desired peptide must accommodate a plurality of factors affecting not only endopeptidase cleavage but polypeptide expression, production and accumulation as well. A fusion protein system applicable to the production of a wide variety of desired proteins or peptides does not currently exist which will satisfy all these factors.

As indicated earlier, one important embodiment of the claimed invention involves production of atrial peptides as a bacterially produced fusion protein, cleavage of the fusion protein and recovery of the product. Mammalian atria contain peptides that exert potent effects on kidney function and regional vascular resistance. These peptides, originally extracted from rat atria and exerting natriuretic, diuretic and smooth muscle relaxant (e.g. vasodilating) activities are currently referred to as atrial peptides.

Rat atrial extracts have been fractionated into low molecular weight fractions (<10,000 daltons) and high molecular weight fractions (20,000-30,000 daltons) both of which relaxed smooth muscle _in vitro_ and were potent natriuretic agents when administered intravenously to rats. See Currie et al. (1983). Trippodo et al. (1982) found natriuretic activity in the overall molecular weight range of 3,600 to 44,000 daltons and in peptide fractions of both higher molecular weight range of 36,000-44,000 daltons and a lower molecular weight in the range of 3,600-5,500 daltons.

Efforts devoted to the purification and chemical characterization of atrial peptides have been hampered by the scarcity of material available from atrial homogenates and by the apparent heterogeneity of the biologically active factor. The amino acid sequence of several atrial peptides is now known, see U.S. Patent No. 4,496,544; U.S. Patent No. 4,508,712; European Patent Application publication number 116,784 (published 8/29/84); Seidah, N.G. et al. (1984); deBold et al. (1983); deBold and Flynn (1983). The low molecular weight nature (<10,000 daltons) of many of the atrial peptides identified to date will undoubtedly require the fusion protein approach for production in bacteria.

Accordingly, it is an object of the present invention to provide endopeptidases useful in achieving optimum cleavage of a bacterially produced fusion protein to release a desired heterologous peptide.

It is another object of the present invention to provide methods for cleaving bacterially produced fusion proteins to achieve the release of desired heterologous peptides in their mature form.

It is yet another object of the invention to provide methods for producing in bacteria atrial peptides having useful natriuretic, diuretic and/or vasodilating activity.

It is a further object of the present invention to provide methods for producing in bacteria, fusion proteins containing atrial peptides which have useful natriuretic, diuretic and/or vasodilating activity.

It is still a further object of the present invention to provide methods for producing in bacteria fusion proteins containing atrial peptides wherein said atrial peptides can be enzymatically released from said fusion protein in their mature form.

It is yet a further object of the present invention to provide fusion proteins which allow for high level of production, in bacteria, of atrial peptides and affords site-specific cleavage and release of atrial peptides from the fusion protein.

## Summary of the Invention

The present invention provides methods for producing a heterologous peptide in bacteria. Also provided are novel DNA sequences encoding heterologous peptides and various genes, DNA vectors and transformed bacteria useful in practicing the method of the present invention. One method involves expressing in bacteria genomic DNA encoding a fusion protein, the fusion protein comprising a heterologous peptide linked to an endogenous protein at a junction site, wherein both the endogenous protein and junction site have an endopeptidase cleavage site; recovering the fusion protein; treating the fusion protein with a suitable endopeptidase such that the endogenous cleavage site at the junction site is preferentially cleaved while the endopeptidase cleavage site in the endogenous protein is substantially intact; and obtaining therefrom the desired heterologous peptide.

The present invention also provides heterologous polypeptides comprising a recA protein or portion thereof, an endopeptidase cleavage site and an atrial peptide.

Heterologous peptides obtainable by the method of the present inventions are atrial peptides. The novel DNA sequences encode atrial peptides.

<u>Brief Description of the Drawings</u>

In the following diagrammatic representations, the directional arrows represent the 5' to 3' orientation of the DNA coding sequences. Relevant restriction endonuclease sites are also shown. The DNA regions so marked are for purposes of diagrammatic representation only and are not drawn to scale.

Figure 1    shows the complete dsDNA sequence encoding APIII prepared for insertion into the vector M13mp9 along with the corresponding amino acid sequence. "1" denotes the start of the mature APIII peptide.

Figure 2    depicts the construction of M13mp9/APIII comprising m13mp9 carrying an APIII DNA coding sequence. The hatched box represents the DNA coding sequence for APIII.

Figure 3    depicts the construction of the synthetic double-stranded APIII DNA coding sequence. The ∧, ∨ symbols show the place where ligation occurs.

Figure 4    depicts the construction of pMON1461 comprising a pBR322 plasmid having inserted therein at the EcoRI/BamHI restriction sites DNA encoding 70% <u>recA</u>.

Figure 5    depicts the construction of pMON2558 comprising a m13mp9 vector having inserted therein at the EcoRI/SmaI restriction site DNA encoding 70-100% <u>recA</u>.

Figure 6   depicts the creation of an EcoRI restriction site at the 3'-end of the 70-100% recA DNA coding sequence by oligonucleotide-directed site-specific mutagenesis.

Figure 7   depicts the construction of pKO1(RI⁻)/M13 linker comprising a pKO1 vector in which the EcoRI restriction site has been removed and having inserted therein at the HindIII/SmaI restriction site a M13mp9 linker carrying a BamHI restriction site.

Figure 8   depicts the construction of pKO1(RI⁻)/XbaI comprising a pKO1(RI⁻)/M13 linker vector having inserted therein at the SmaI restriction site an XbaI restriction site.

Figure 9   depicts the construction of pMON6150 comprising a pKO1(RI⁻)/XbaI vector having inserted therein at the XbaI restriction site a DNA sequence encoding APIII and having inserted therein at the BamHI restriction site a DNA sequence encoding 70% recA.

Figure 10  depicts the construction of pMON6152 comprising a pMON6150 vector having inserted therein at the EcoRI restriction site a DNA sequence encoding 70-100% recA.

Figure 11  depicts the construction of pMON6154 comprising a pUC18 vector having inserted therein at the BamHI restriction site DNA encoding the recA-Glu-APIII gene.

Detailed Description of the Invention

The present invention provides a method for obtaining heterologous peptides from fusion proteins by site-specific cleavage with an endopeptidase. Suitable heterologous peptides include eucaryotic hormones, one example of which are atrial peptides. Suitable endopeptidases include, but are not limited to, Trypsin, Plasmin, Enterokinase, Kallikrein, Urokinase, Tissue Plasminogen Activator, Clostripain, Chymotrypsin, Pepsin, Chymosin, Collagenase, Russell's Viper Venom Protease, Post-proline cleaving enzyme, Staphyloccus aureus strain V8 extracellular protease, herein referred to as "V8 protease", blood coagulation factor Xa, herein referred to as "factor Xa," and thrombin, wherein V8 protesase, thrombin and factor Xa are the preferred endopeptidases.

V8 protease is an endopeptidase which cleaves specifically the peptide bonds on the carboxy-terminal side of either aspartate (Asp) or glutamate (Glu) residues. See Houmard and Drapeau (1972a and b). Due to its single amino acid specificity, V8 protease has been used extensively in peptide mapping. See McWhereter, C. A. et al. (1984); Hausinger and Howard (1982); Johnson, J. S. (1983); Cleveland et al. (1977). In the present description, the term V8 protease refers to any V8 protease derived from a bacterial or any recombinant or synthetic source.

V8 protease has two pH optima, pH 4.0 and 7.8, and is active in both urea and sodium dodecyl sulfate (SDS), reagents often employed to solubilize proteins. These cleavage properties render V8 protease attractive for use in cleavage of bacterially produced fuison proteins.

One study by Austen and Smith (1976), which examined the ability of V8 protease to cleave Glu when this residue was at or near either the N- or C-terminus of a peptide indicated that no cleavage occurs when Glu is within two residues of the N-terminus or within two to three residues from the C-terminus. The authors interpreted these findings as suggesting that the peptide must be bound to the enzyme over a region extending several residues on either side of the potentially susceptible bond. The single observed cleavage at Glu when the Glu residue was within two residues of the N-terminus was attributed by these authors to be possibly due to the increased polar character of the residues on the C-terminal end of the peptide studied. V8 protease enzymatic activity, however, is generally believed not to be affected either adversely or positively by neighboring amino acid residues with the possible exception of Asp-X wherein X is cysteic acid, and Glu-X wherein X is glutamic acid or proline which have not been observed to be cleaved by V8 protease. See Houmard and Drapeau (1972a) and (1972b); Behrens and Brown (1976); Austen and Smith (1976).

Employment of V8 protease in fusion protein cleavage systems, however, necessarily requires that the desired peptide not contain any trigger amino acids, herein Glu or Asp, and/or that any internal Glu or Asp residues be unavailable for V8 protease cleavage under the cleavage conditions employed. It is additionally desirable that the endogenous protein not contain any trigger amino acids and/or that any internal Glu or Asp residues be unavailable for V8 protease cleavage under the cleavage conditions employed.

Blood coagulation factor Xa is a member of the serine protease group. In vivo, factor Xa activates prothrombin to thrombin by specific limited proteolysis at the bonds Arg (274)-Thr (275) and Arg (323)-Ile (324). In prothrombin, both cleavage sites are preceded by the same tetrapeptide, Ile-Glu-Gly-Arg, which has been proposed as a determinant of factor Xa substrate recognition. (Magnusson, S. et al., 1975). Some of the peptide sequences known to be cleaved by factor Xa are also described in EPA publication number 161,939 (published 11/21/85). It appears that the structure required for recognition by factor Xa is determined by the local sequence at the cleavage site (EPA 161,939).

The DNA sequence of the present invention may code for any cleavage site which is specifically cleaved by factor Xa including all of the specific factor Xa cleavage sequences (i.e. trigger signals) previously described. In one preferred embodiment, a novel factor Xa trigger signal is described comprising the sequence $NH_2$-Phe-Glu-Gly-Arg-COOH.

As is the case with V8 protease and thrombin, discussed below, the specific nucleotides present in the gene coding for a fusion protein containing a factor Xa trigger signal will depend upon the particular amino acid sequence of the trigger signal and the genetic code. Thus, in view of the redundancy (i.e. degeneracy) of the genetic code, a plurality of different DNA sequences can be used to code for a single trigger signal. A particular DNA sequence can be chosen having regard to host codon usage preferences and/or to facilitate DNA manipulations, for instance to provide convenient restriction endonuclease sites.

Treatment of proteins including fusion proteins with factor Xa results in substantially exclusive cleavage at the peptide bond following the arginine (Arg) amino acid residue of the factor Xa trigger signal to liberate a protein fragment or desired peptide in native form. The term "native form" refers to a polypeptide or peptide comprising the amino acid sequence thereof without additional amino acid residues, e.g. an N-terminal methionine or N-terminal host protein amino acid residues.

Thrombin is a serine proteinase that catalyzes the cleavage of certain arginyl and lysyl bonds. Thrombin has been found in the blood plasma in all classes of vertebrates and is an important enzyme in blood coagulation. The naturally occurring substrate for thrombin is fibrinogen which is cleaved by thrombin at an arginylglycyl bond to form fibrin. (Magnusson, S., 1971). Trigger signals thus far identified for thrombin include Glu-Gly-Arg. (Magnusson, 1971).

The choice of which endogenous protein sequence or fragment thereof to employ in construction a gene for production in bacteria of a fusion protein containing the desired peptide depends upon several factors. A "gene" is defined herein as a DNA sequence encoding the protein product to be expressed and the necessary transcription and translation control signals for production of the desired protein product in a given host cell. These factors include, but are not limited to, the availability of the chosen endogenous gene sequence or fragment thereof, the strength of the attendant promoter, facility with which the expression of said gene may be induced and/or controlled in a given host cell, size of the expressed endogenous product or fragment thereof, the ability to solubilize the fusion protein produced in a given host to accommodate subsequent cleavage and release of the desired peptide and/or ability to monitor expression of the endogenous DNA sequence. Additionally, the chosen endogenous protein or fragment thereof should yield both a stable and high level accumulation of the desired protein or peptide product when joined thereto to yield a fusion protein. Furthermore, the resultant fusion protein must be either soluble or solubilizable so that subsequent site-specific enzymatic release of the desired protein or peptide can be achieved.

A number of bacterial genes which are readily expressed at high levels in bacteria are known to those skilled in the art and include, without limitation, the chloramphenicol acetyl transferase (CAT) gene, the β-galactosidase gene (lac Z) and recA gene. Such genes exemplify endogenous genes which can be used as fusion carriers with the desired protein or peptide product.

In one embodiment, the recA gene or fragments thereof was employed as a fusion carrier for production of such peptides as atrial peptides. The use of a recA gene to affect production of atrial peptides by means of a fusion protein comprising recA or a fragment thereof and an atrial peptide has heretofore not been disclosed. Feinstein, S. et al. (1983) describes the use of a recA promoter to directly express in bacteria human interferon species and the use of a gene construct comprising, in part, a recA promoter, ribosome binding site and first three codons of the recA DNA coding sequence to produce in bacteria a recA-β-interferon "fusion-like" protein. The term "fusion-like" protein is used to denote the limited amount of recA protein (e.g. three amino acids) present in the β-interferon containing product protein. EPA publication number 108,045 (published May 9, 1984) and commonly assigned to Monsanto Company, describes the use of a recA promoter/operator to directly express somatostatin in bacteria. This EPA published application also describes the production in bacteria of a recA-somatostatin fusion protein comprising either 70% or 90% of the recA protein and 100% of the somatostatin peptide. We have determined that both the 70% and 90% recA fusion constructs are inoperative for production in bacteria of recA-atrial peptide fusion proteins as the products produced in accordance with these teachings yield an insoluble fusion protein hence precluding subsequent enzymatic cleavage to release the desired atrial peptide.

None of the previously described art teaches or suggests an economic and efficient means for producing large quantities of atrial peptides in bacteria for both scientific and therapeutic uses.

The selection of the recA protein as an endogenous protein carrier in the fusion protein systems described herein was also made based upon certain advantageous physical properties of the recA protein. Specifically, the recA protein is a highly negatively charged protein. The highly charged nature of the recA protein affords a major advantage in the subsequent purification of bacterially produced fusion proteins containing recA or portions thereof which retain the net negative charge. For example, such conventional methodologies as anion exchange chromatography can be employed to isolate recA proteins from substantially all other bacterial proteins.

In one embodiment of the present invention, V8 protease was chosen to specifically release an atrial peptide from a bacterially produced fusion comprising the entire recA protein of bacteria joined to an atrial peptide by a V8 protease-specific trigger signal. Atrial peptides contain no glutamic acid residues and only one aspartic acid at position nine. See U.S. Patent No. 4,496,544. The recA protein, however, contains numerous (i.e. approximately 31) glutamic acid residues.

Notwithstanding the presence of an internal Asp residue in the desired peptide and numerous Glu residues in the recA protein, we proceeded to attempt the specific release of an atrial peptide from a bacterially produced recA-atrial peptide fusion protein. "Internal" is herein defined as any amino acid not at the N- or C-terminus of the protein. In so doing, we discovered that V8 protease preferentially cleaves a Glu trigger signal sequence present at the junction site, thereby promoting an early release of the desired peptide prior to cleavage of internal Glu residues present in the recA protein.

The present discovery is significant since it provides a method for specifically cleaving recA-atrial peptide fusion proteins containing a Glu residue in a trigger signal present at the junction site. Furthermore, the observed early release of the desired peptide from said fusion protein represents an unexpected result as one would have predicted a simultaneous cleavage at Glu residues within the recA protein thereby masking the specific release of the desired atrial peptide, and possible impeding subsequent isolation of the desired atrial peptide essentially free from the recA protein or fragments thereof. Indeed, cleavage at the internal Glu (V8 protease) sites does eventually occur if the V8 protease cleavage reaction is allowed to proceed to completion. The methods described herein thereby provide a means for specifically or preferentially releasing such desired peptides as atrial peptides from bacterially produced fusion proteins comprising recA. The endogenous protein (e.g. recA) is thus defined herein as remaining substantially intact wherein the desired heterologous peptide can be distinguished from endogenous protein or fragments thereof following endopeptidase cleavage of the fusion protein.

While applicants do not wish to be bound by the following theory of mechanism, it is believed that the conformation of the bacterially produced recA-containing fusion proteins renders the V8 protease trigger signal present at the junction site available for endopeptidase cleavage while, at least initially, rendering internal trigger amino acids unavailable for cleavage by V8 protease. It is understood that the unavailability of the internal recA trigger amino acids can result from such structural features as stearic hinderance and/or from kinetic properties associated with the recA-V8 protease interactions. It is furthermore believed that such selective availability of V8 trigger amino acids at the junction site will result from any heterologous peptide fused to a rec A protein or fragment thereof by a junction site containing a V8 protease trigger amino acid.

In one of the preferred embodiments, the method of the present invention is employed to generate atrial peptide I, III or IV (API, APIII or APIV, respectively) (see U.S. Patent No. 4,496,544 and EPA publication number 116,784) from a bacterially produced recA-containing fusion protein, free from other atrial peptide species. The ability to produce a single atrial peptide species has tremendous import for determining the precise bioreactivity of said species and for obtaining commercial quantities of atrial peptides. Additionally, once the bioreactivity of such atrial peptide species is determined, it is considered feasible to generate peptide variants which would further increase their bioreactivity. Such variants can be generated by nucleotide or amino acid deletion, substitution and/or addition in accordance with techniques described herein and known to those skilled in the art. The production of atrial peptide variants by the method of the present invention are considered to be within the scope of the appended claims.

In its broadest embodiment, the present invention is a refinement in the use of recombinant DNA technology to produce heterologous peptides in bacteria. Thus, the description of the present invention presupposes knowledge of the basic techniques employed in recombinant DNA technology to isolate and clone DNA sequences encoding peptides and proteins, the rearrangement or altering of cloned DNA sequences, and the expression of cloned or modified DNA sequences in transformed microorganisms. Such techniques are within the skill of the art. See e.g. Maniatis et al. (1982).

Isolation and Construction of Heterologous DNA

Production of heterologous (e.g. eucaryotic) peptides in bacteria requires the isolation or synthesis of a DNA sequence encoding the desired peptide. Procedures for isolating DNA sequences and for either chemically or enzymatically synthesizing DNA sequences are well known to those skilled in the art.

It has been found that both E. coli and yeast exhibit various codon preferences. See Craig, R. K. and Hall, L. (1983); Fiers, W. et al. (1976); Ikemura, T. (1982). Thus, to achieve optimum translation of a heterologous mRNA sequence, it may be desirable to substitute those codons preferred by the host cell employed.

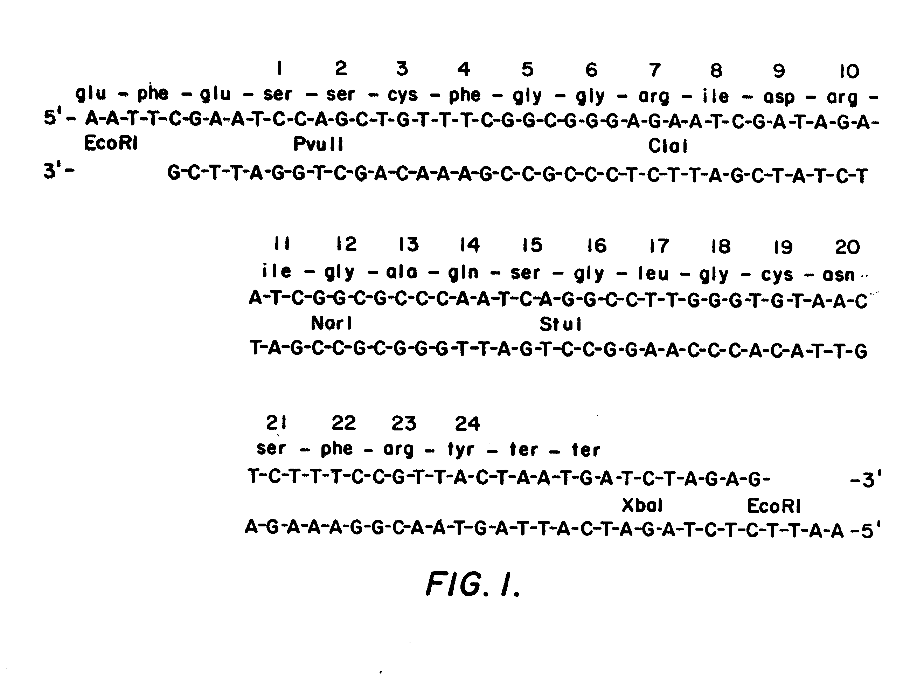

In a preferred embodiment of the present invention, a novel DNA sequence encoding APIII was constructed containing preferred codons of both E. coli and yeast cells which, furthermore, allow introduction of restriction endonuclease cleavage sites useful in the manipulation and screening of this DNA sequence. Briefly, a novel DNA sequence encoding the 24 primary amino acid structure of atrial peptide III (APIII) was synthetically produced, as described more fully below. The novel DNA coding sequence of the 72 base pairs (bp) was constructed taking into account both E. coli and yeast host cell codon preferences. The coding part of the sequence was preceded by a codon for glutamic acid providing a recognition site for cleavage of the peptide from a fusion protein with V8 protease. The coding part of the sequence was immediately followed by at least one translation termination codon. The recognition sites for EcoRI and XbaI restriction endonucleases were introduced into the polynucleotide sequence at sites indicated in Figure 1 to facilitate recombinant manipulation and subsequent monitoring of the synthetic DNA. Thus, the inclusion of these restriction sites is optional. Similarly, alternate and/or additional restriction endonuclease sites may be introduced.

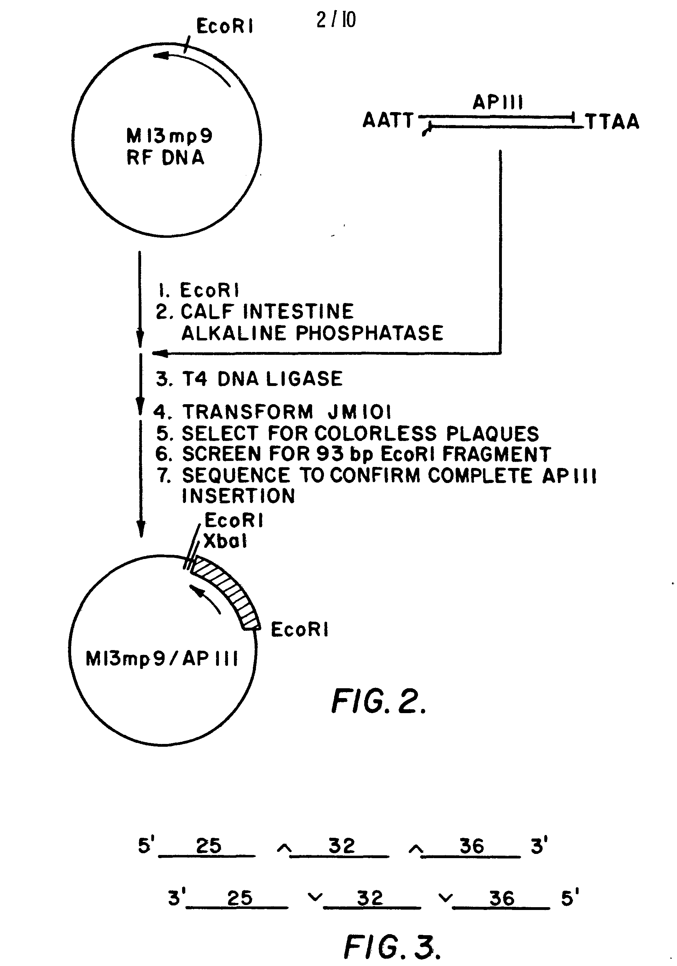

In order to assemble a double-stranded DNA (dsDNA) fragment encoding the APIII peptide shown in Figure 1, six complementary and partially overlapping synthetic oligonucleotides were synthesized and subsequently annealed to form a 93 bp oligonucleotide under appropriate conditions, as described more fully below and shown in Figure 3.

After a heterologous DNA sequence containing the codons for the desired polypeptide is obtained, it may be desirable to make certain modifications in the nucleotide sequence of the molecule. For example, if the molecule has been produced by reverse transcription from a messenger RNA (mRNA) template, in lieu of chemical synthesis, it will often contain at least a portion of the DNA encoding the leader sequence of the pre-protein. Thus, it may be necessary to remove all of the leader sequence DNA prior to the first codon of the desired protein.

If not already present, at least one translation stop signal is introduced after the codon for the C-terminal amino acid of the desired peptide. Examples of translation stop signals include the deoxynucleotide triplets (i.e. codons) TAA, TGA and TAG.

As described below, recombinant DNA techniques and/or chemical synthesis were employed to construct a heterologous DNA sequence containing sequentially a glutamic acid codon or the codons for a factor Xa or thrombin trigger signal, the codons for the desired peptide and at least one translation stop signal codon adjacent to the codon for the C-terminal amino acid of the desired peptide.

In constructing the desired heterologous DNA coding sequence, deletions, additions and/or substitutions in any of the amino acid codons within a given heterologous DNA sequence may be made so that a "variant" peptide can be expressed in the process of the present invention. A variant peptide is defined herein as having single or multiple amino acid deletions, substitutions and/or additions as compared to the naturally occurring amino acid sequence of a given peptide. Because these variant peptides have an amino acid sequence essentially the same as that of a naturally occurring peptide, their biological activity is not diminished to an intolerable degree. Construction and expression of variant peptides may be desirable in order to achieve increased fusion protein accumulation, increased peptide and/or fusion protein stability, to facilitate peptide and/or fusion protein purification, and/or to optimize biological activity.

The above modifications of the DNA molecule encoding the desired polypeptide can be accomplished using restriction enzymes, exonucleases, endonucleases, etc. by techniques known in the art. See Maniatis, et al. (1982). The general techniques of oligonucleotide-directed site-specific mutagenesis can also be employed to effect the above modifications in the structure or sequence of the DNA molecule and are known to those of skill in the art. See Zoller & Smith (1982); Zoller & Smith (1983); Norris et al. (1983).

After multiple copies of the desired heterologous DNA sequence are obtained, these sequences may be removed from the recombinant vectors and inserted into an expression system for production and isolation of the desired heterologous peptide as described more fully below. Modifications of the heterologous DNA sequence, by methods known to those skilled in the art, may be made prior to insertion of these DNA sequences into an expression vector, during said insertion and/or following said insertion.

As previously described, production of fusion proteins by bacteria is achieved by the site-specific insertion of the DNA sequence encoding the desired peptide into or immediately downstream from the DNA encoding an endogenous protein or fragment thereof under the control of a bacterial promoter where the endogenous DNA sequence and promoter are carried on an expression vector. Thus, the desired heterologous gene contains DNA sequences encoding an endogenous protein or fragment thereof, an endopeptidase trigger signal and the desired heterologous peptide.

As previously discussed, the choice of which endogenous gene to employ depends on several factors such as the ability to produce in the host cell a highly expressed, soluble protein when coupled with the desired heterologous peptide.

In one preferred embodiment of the present invention, the recA gene of E. coli carried on an expression vector was used as the endogenous DNA sequence to which the desired peptide DNA coding sequence was fused. The recA gene of E. coli is involved in important cellular functions such as genetic recombination, in post replication repair, and in a number of other cellular functions such as mutagenesis, phage induction and cell division. See Sancar, A. and Rupp, D. (1979); Witkin, E.M. (1976). Functional and/or chemical homologs of the E. coli recA protein have been described in other bacterial genuses such as Proteus (e.g. Proteus vulgaris), Erwinia (e.g. Erwinia carotovora) and Shigella (e.g. Shigella flexneri), Keener, S. et al. (1984). A recA "homolog" is herein understood to comprise a protein, the gene of which is contained within the genome of bacteria other than E. coli, and which protein is characterized as possessing the following characteristics: it functions as a DNA repair enzyme (e.g. an ability to restore resistance to ultra-violet killing in recA$^-$ E. coli), and/or shares substantial DNA or amino acid sequence homology with the E. coli recA protein and/or is a highly negatively charged protein having a conformation which allows preferred cleavage at a junction site comprising a V8 protease trigger signal. These homologs which possess substantially the same physical-chemical and/or functional properties of the E. coli recA protein are believed to constitute equivalents of the E. coli recA protein specifically described herein. (Keener, S. et al. 1984).

The recA gene of E. coli is normally repressed by the product of the lexA gene but can become induced by treatments with substances such as naladixic, mitomycin C or ultra violet radiation, all of which damage DNA. Treatment of E. coli with these substances stimulates the recA protein, which is present at low, residual levels, to cleave its repressor and undergo induction. After induction, the recA protein becomes one of the major proteins in the cell, indicating that the combination of a recA promoter and ribosome binding site is an efficient one for inducible expression. See Feinstein, S. et al (1983). The inducible nature of the recA promoter is advantageous in cases where constitutive expression of a cloned gene at such high levels would be harmful to the cell or undesirable for purposes of optimizing purification of a desired recA fusion protein.

Furthermore, any bacteria strain which is recA$^+$ can be employed for production of recA fusion proteins. Such bacteria include Proteus mirabilis (Eitner et al., 1982) and E. coli wherein E. coli are preferred host organisms. The more preferred strains of E. coli are both recA$^+$ and lexA$^+$ as these strains provide for an inducible control of recA fusion protein production.  Examples of such preferred E. coli host strains include E. coli MM294 and E. coli JM101, wherein E. coli JM101 constitutes the most preferred host cell for recA fusion protein production.  Both of these strains may be obtained from the American Type Culture Collection (ATCC), Rockville, Maryland under ATCC accession numbers 33625 and 33876, respectively.  Thus, when employing a fusion protein expression vector which codes for a recA homolog, equivalent bacterial host cells selected from, for example, such gram negative bacteria as Enterobacteriaceae, Erwinia, Shigella, Salmonella and Klebsiella, containing a gene for the recA homolog can be employed.  The more preferred host cells would contain functional genes for both the recA homolog and recA homolog repressor.

We have discovered that the production in bacteria of a soluble recA-atrial peptide fusion protein requires employment of the entire (i.e. 100%) recA DNA coding sequence.  This finding is significant as prior descriptions of the employment of the recA promoter and DNA coding sequences have suggested that use of greater than 70% of the recA DNA coding sequence yields an insoluble fusion protein product in bacterial cell hosts.  See EPA 108045 (published May 9, 1984).

The method of the present invention, described more fully below, provides a means for producing in bacteria high levels (e.g. approximately 10 to 30% of the total cell protein) of a stably accumulated heterologous peptide (e.g. atrial peptide) which can be purified to yield a potent natriuretic and/or smooth muscle relaxant agent.

## Cloning of Heterologous DNA

In accordance with recombinant DNA techniques, once the desired heterologous DNA sequence is obtained, the sequence is then inserted into an appropriate cloning vector which provides a means for replicating the DNA sequence. Any appropriate cloning vector, preferably containing a marker function, may be used, for example E. coli plasmid vectors which include Col El, Hershfield et al. (1974); pBR322, Bolivar et al. (1977); pBR325, Soberon et al. (1978); and pkc7, Rao et al. (1979); and E. coli bacteriophase vectors which include Charon AL47.1, Loenen et al. (1980); and M13mp8 and M13mp9, Messing et al. (1982). The general techniques for inserting said DNA sequence into a cloning vector to create a recombinant vector are within the skill of the art. See Maniatis et al. (1982).

In the examples of the present invention, M13mp8 and M13mp9, described by Messing et al. (1982), and pUC18, pBR327, pBR322 and pKO1 were chosen as the cloning vectors.

The M13mp9 and M13mp8 vectors, hereinafter referred to as the M13 vector or vectors, allow for isolation of recombinant vectors in both double-stranded (ds) or replicative form (RF) and single-stranded (ss) DNA forms. Isolation of RF DNA recombinant vectors facilitates the subsequent insertion of the replicated desired DNA sequences into expression vectors. Alternatively, isolation of the single-stranded form of these recombinant vectors facilitates isolation of recombinant vectors which contain the desired DNA sequence in a proper 5' to 3' orientation for expression, the creation of any DNA sequence modification by such techniques as

07-21(355)A

oligonucleotide-directed site-specific mutagenesis, and facilitates DNA sequence analysis. Additionally, these M13 vectors can accommodate DNA fragments or genes up to 4kb in length which insures the cloning of a typical, entire, eucaryotic gene sequence.

The marker function employed in the M13 vector, as described by Messing et al. (1982), involves the enzyme for β-galactosidase. Specifically, the desired heterologous DNA sequence is inserted into the linker preceding the lacZ gene fragment carried on the M13 vector which disrupts the normal complementation of the lacZ gene carried on the M13 vector with the partial lacZ gene fragment carried on the chromosomal DNA of the host cell (e.g. E. coli JM101) so that said host is no longer capable of metabolizing lactose present in the bacterial growth medium. E. coli infected with M13 vectors which do not have a foreign gene sequence inserted into the vector born lacZ gene fragment are capable of metabolizing lactose present in the bacterial growth medium and yield characteristic blue plaques when the bacteria are grown on agar containing 1 x YT medium comprising 0.8% (w/v) tryptone, 0.5% (w/v) yeast extract, 0.5% (w/v) NaCl and a color indicator for β-galactosidase. The plaque coloration of E. coli infected with recombinant vectors carrying an inserted heterologous DNA sequence in the M13 lacZ gene fragment is clear or colorless when the bacteria are grown on said medium. Hence, positive insertion of the heterologous DNA sequence into these cloning vectors is identified by colorless plaque formation following infection of the E. coli host cell with the recombinant vector.

The marker functions on the pBR322, pBR327, pUC18 and pK01 cloning vectors are identified by a preliminary screen for antibiotic resistance and/or colony coloration, as described more fully below, and then restriction endonuclease analysis to confirm the insertion of the desired DNA coding sequences into the vectors, as described more fully below.

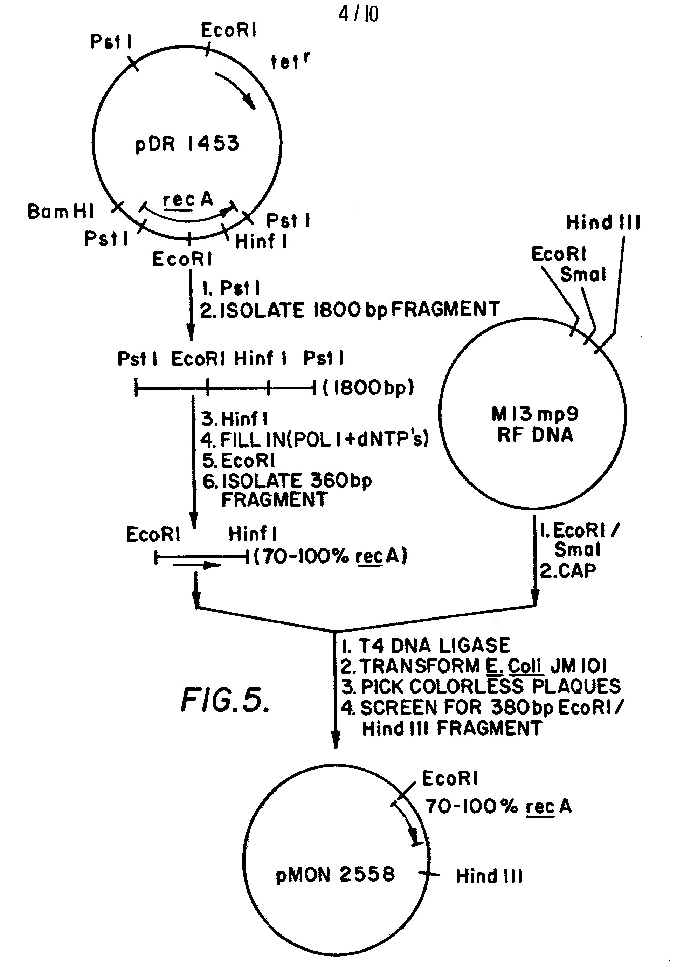

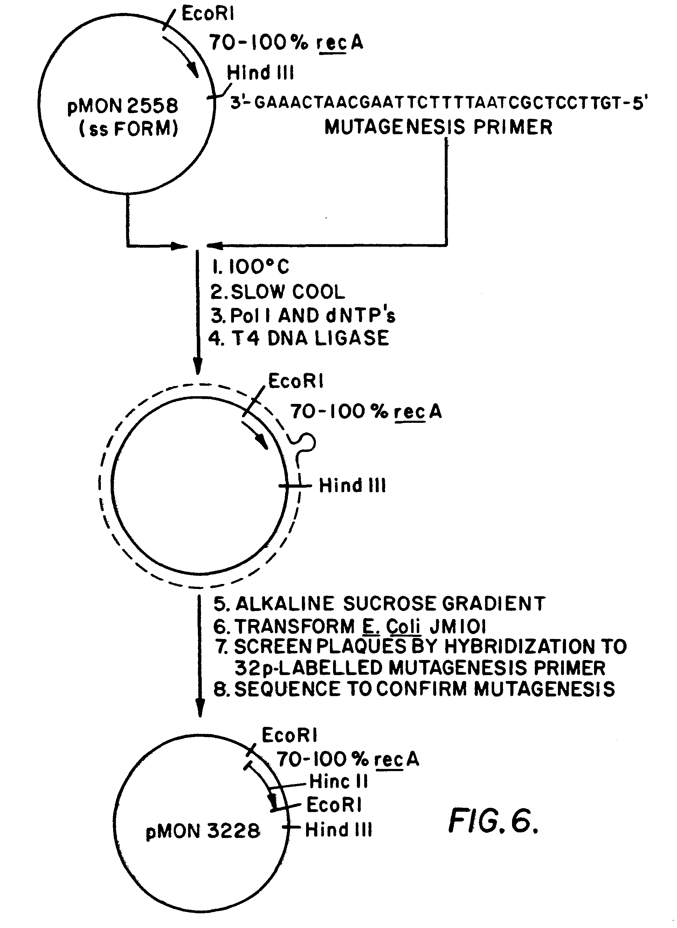

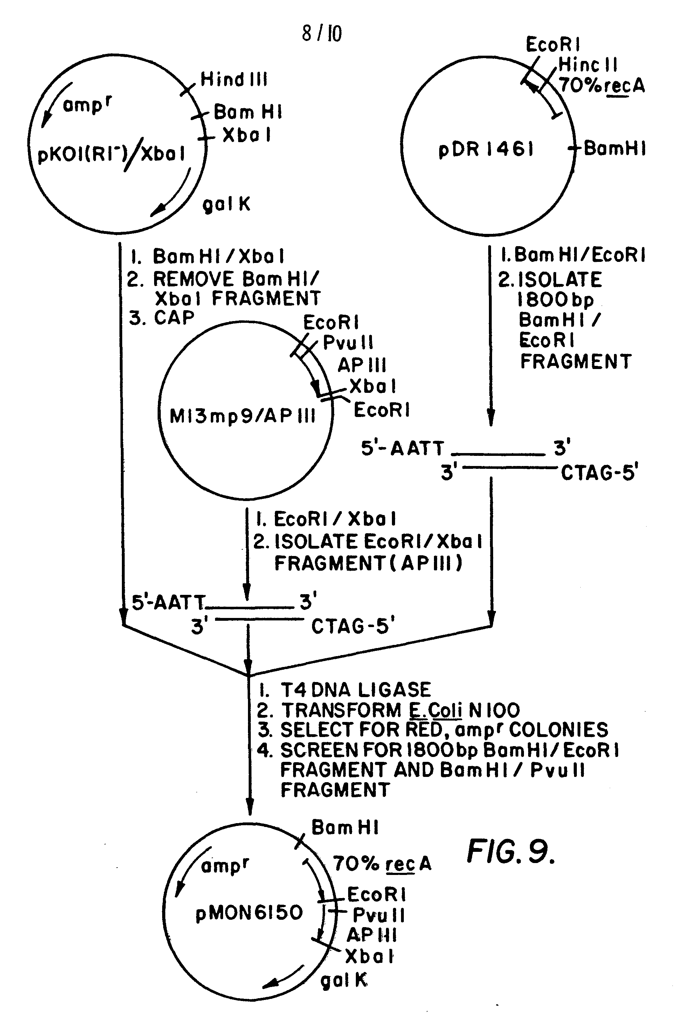

In a preferred embodiment, DNA encoding the C-terminal 70-100% of the recA protein, herein referred to as 70-100% recA, was inserted into RF DNA of the M13 vector as shown in Figure 5 to create a recombinant cloning vector, pMON2558, as described more fully below in the examples. Oligonucleotide-directed site-specific mutagenesis was then employed as shown in Figure 6 and described more fully below to introduce an EcoRI restriction site at the 3'-end of the recA DNA coding sequence to create recombinant cloning vector pMON3228. Said site was introduced to facilitate subsequent cloning of the 70-100% recA to create expression vector pMON6152 as shown in Figure 9.

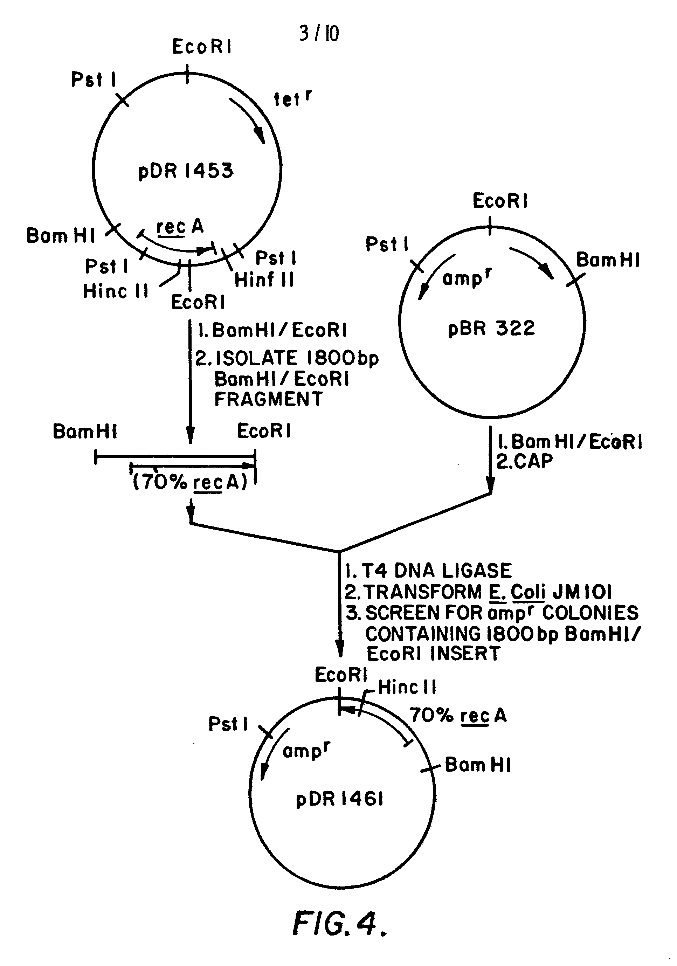

The DNA encoding the recA promoter, ribosome binding site and N-terminal 70% of the recA gene product, collectively referred to herein as 70% recA, was isolated as shown in Figure 4 below and cloned into a pBR322 cloning vector to create recombinant cloning vector pDR1461 as shown in Figure 4 and as described more fully below.

In a preferred embodiment, the synthetic APIII DNA coding sequence shown in Figure 1 was inserted into the unique EcoRI site in M13mp9 to create an APIII containing recombinant cloning vector as shown in Figure 2. The APIII insertion was confirmed by transfecting E. coli JM101 with the recombinant cloning vector in accordance with the method described by Messing et al. (1983), selecting colorless plaques and then isolating single-stranded recombinant phage DNA in accordance with the method described by Messing et al. (1983), the relevant portions of which are incorporated by reference hereto.  The single-stranded phage DNA was then sequenced by the dideoxy chain termination method described by Sanger et al. (1977) to verify the insertion of the complete APIII DNA coding sequence.

Subsequent to cloning the DNA sequences encoding the desired portions of a preferred fusion gene, these sequences can be replicated and numerous copies generated by propagation of the respective recombinant cloning vector by methods known to those skilled in the art and referenced above.  These heterologous DNA sequences can be inserted into any appropriate expression vector for production in bacteria of the desired heterologous polypeptides (e.g. fusion proteins).

### Production of Fusion Proteins Containing Glu Trigger Signal

As described previously, an appropriate expression vector should contain the necessary transcription and translation signals for production of a heterologous protein in the chosen host cell along with a marker function for identification of those expression vectors into which the desired heterologous DNA sequence has been inserted.  By use of a procaryotic expression vector, the recombinant DNA sequences can be added to the genetic complement of a procaryotic organism via transduction, transformation or transfection (collectively referred to herein as "transformation") and the organism can then be cultured under conditions (generally governed by the promoter and host employed) that cause the desired polypeptide to be produced. Thus, the "genomic" DNA of the organisms used in this invention contains both chromosomal and episomal DNA.

A number of expression vectors have been described for heterologous gene expression and heterologous protein production in procaryotic host cells and are known to those skilled in the art. These expression vectors include expression systems in which the promoter contained therein provides for either constitutive or inducible gene expression.

In one preferred embodiment of the present invention, a pK01 vector obtained from Dr. Martin Rosenberg, National Institutes of Health (Bethesda, Maryland) and described in Chirikjian, A. and Papas, T. (1981) was employed as an expression vector. pK01 is a pBR plasmid derivative which carries an E. coli galactokinase gene. The galactokinase gene (galK) product is readily expressed and stably accumulated in E. coli N100 transformed with a pK01 plasmid in which a promoter has been inserted upstream from the galK gene and can serve as a marker for transformation by a number of assays. Rosenberg et al. (1968). E. coli transformed with pK01 form red colonies on galactose MacConkey agar whereas non-transformed colonies are white. Alternatively, the gene product may be identified as a discrete band when transformed E. coli lysates are subjected to polyacrylamide gel electrophoresis (PAGE) or by an enzymatic assay analysis by assaying for the phosphorylation of galactose.

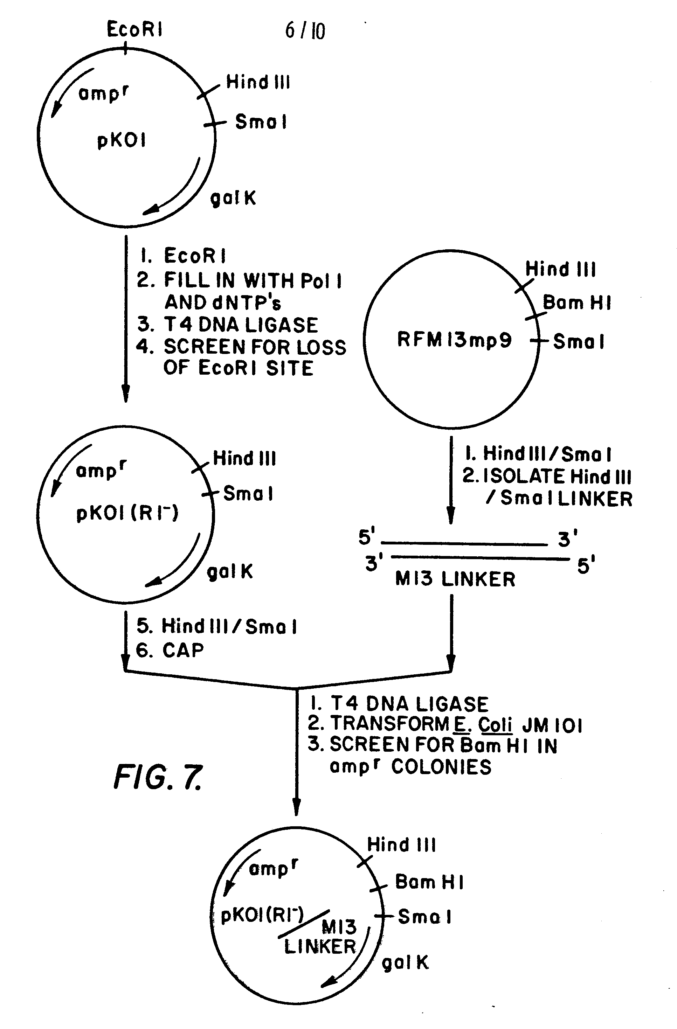

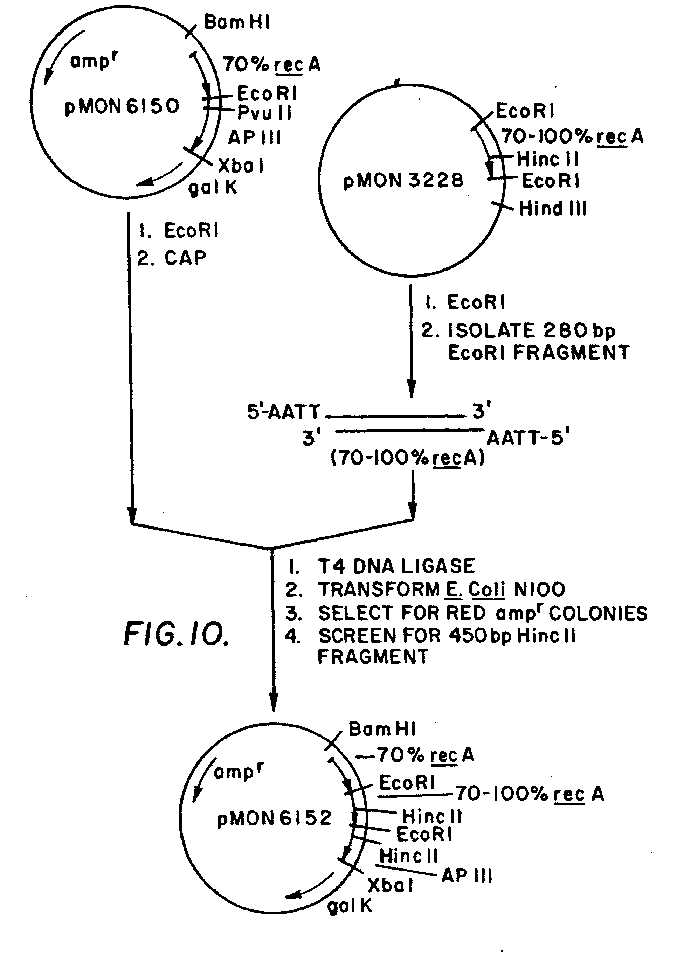

In an example of the present invention, expression vector pMON6152 comprising pK01 carrying a complete recA gene and APIII DNA coding sequence was created as shown in Figs. 7-10.  A bacteria such as E. coli N100 was then stably transformed with the pMON6152 expression vector and transformants selected by growth on galactose MacConkey agar-containing 200 µg/ml ampicillin as described more fully in the examples.  The expression plasmids contained within the transformed bacteria were then screened for the presence of the recA and APIII coding sequences in the correct 5' to 3' orientation as shown in Fig. 10 by restriction enzyme cleavage.

Purification of the fusion protein produced will depend on both the protein and host cell chosen. Subsequent purification of the fusion protein to rid it of contaminating bacterial proteins can be achieved by conventional chromatographic means such as gel filtration, ion exchange chromatography or protein-specific (e.g. antibody or substrate) affinity chromatography.  A detailed purification of recA-containing heterologous proteins (e.g. fusion proteins) employing a recA-specific monoclonal antibody described in a concurrently filed U.S. patent application by G. G. Krivi and M. L. Bittner entitled "Antibody Purification of recA Fusion Peptides" and having U.S. Serial No. 747,126, incorporated by reference hereto.  This concurrently filed U.S. patent application and the present application are commonly assigned to Monsanto Company. Additionally, the rec A protein and fragments thereof are highly negatively charged and as such are amenable to purification by such methods as anion exchange chromatography as described more fully in the examples below.

## Fusion Protein Cleavage

Although some investigators have reported that V8 protease hydrolyzes only glutamoyl bonds in either ammonium bicarbonate (pH 7.8) or ammonium acetate (pH 4.0) buffer and hydrolyzes both aspartamoyl and glutamoyl bonds in phosphate buffer (pH 7.8) or sodium acetate buffer (pH 4.0), Houmard and Drapeau (1972a and b), other groups have found no difference in aspartamoyl and glutamoyl cleavage when either ammonium or phosphate buffers are employed. See Behrens and Brown (1976); Austen and Smith (1976). We have determined that the reduced form of a synthetic APIII when subjected to V8 protease digestion in ammonium bicarbonate buffer (pH 7.8) results in cleavage. Misono, D. S. et al. (1984) have reported cleavage of the aspartamoyl bond with V8 protease in sodium phosphate buffer (pH 7.8) in four distinct atrial peptides possessing both natriuretic activity and smooth muscle relaxant activity.

In a particularly preferred embodiment of the present invention, the purified oxidized fusion protein was subjected to V8 protease hydrolysis in an ammonium bicarbonate buffer (pH 7.8) as described more fully below. This buffer was chosen to potentially optimize site-specific cleavage at the junction glutamoyl bond. It has been found that by employing the methods of the present invention, one can achieve from about 80% to 90% release of the desired atrial peptide from the bacterially produced fusion protein.

As described more fully in the examples below, in the process of describing the novel release of atrial peptides from novel bacterially produced fusion proteins, we discovered that V8 protease preferentially cleaves a Glu-containing trigger signal present at the junction site thereby promoting early release of the desired peptide prior to cleavage of internal Glu residues present in the recA protein. We have furthermore discovered that fusion proteins comprising recA joined to an atrial peptide by a glutamic acid molecule promote an early release of a desired heterologous peptide as a single species when a recA-atrial peptide fusion protein is cleaved with V8 protease under appropriate reaction conditions as described more fully below.

Once the fusion proteins have been purified, the proteins are then subjected to endopeptidase cleavage to release the desired peptide. Alternatively, endopeptidase cleavage may be performed on crude extracts from bacteria producing the desired fusion protein and the desired peptide thereafter isolated and purified by conventional chromatographic means such as gel filtration, ion exchange chromatography or protein-specific (e.g. antibody or substrate) affinity chromatography.

In a particularly preferred embodiment of the present invention, the atrial peptide species produced and isolated as described above are shown to exhibit smooth muscle relaxant biological activity as assayed in accordance with methods described by Currie et al (1983) and set forth in U.S. patent number 4,4986,544, the relevant portions of which are incorporated herein by reference.

Briefly, bioassays for the smooth muscle relaxant activity of the isolated peptide were made on rabbit aorta strips or on segments of chick rectum under physiologically acceptable conditions. See Currie et al (1983) and U.S. patent number 4,496,544. Rabbit aorta strips maintained in tone by a continuous infusion of norepinephrine provide a reliable and sensitive assay tissue for measuring the intensity and duration of relaxation by atrial peptides. Natriuretic activity of the isolated peptides may be determined by injecting intravenously in dogs and determining the effect on fractional sodium excretion in the urine. See White and Samson (1954); Pitts, R. F. (1974).

As previously discussed, the invention also contemplates the production and release of various atrial peptide species, variants thereof and other peptides produced as fusion proteins by bacteria. Such atrial peptide species and variants thereof having desirable natriuretic and/or smooth muscle relaxant activities can be identified by routine testing in the biological assays described above.

The following examples illustrate preferred embodiments of the present invention and are not intended to limit the invention's scope in any way. While this invention has been described in relation to its preferred embodiments, various modifications thereof will be apparent to one skilled in the art from reading this application.

### Microorganisms, Plasmids and Materials

The following microorganisms have been deposited with the American Type Culture Collection (ATCC), 12301 Parklawn Drive, Rockville, Maryland, 20852, U.S.A.: ATCC 53147 - E. coli JM101 (pDR1453).

This deposit is available to the public upon the grant of a U.S. patent. This deposit will be available for the life of any such U.S. patent having the benefit of the filing date of this application. However, it should be understood that the availability of a deposit does not constitute a license to practice the subject invention in derogation of patent rights granted by governmental action. Furthermore, the present invention is not to be limited in scope by the microorganism deposited, since the deposited embodiment is intended only as a specific illustration of the invention.

### Example 1

All oligonucleotides were synthesized in the Department of Biological Sciences, Monsanto Company, employing a 380A Applied Biosystems DNA synthesizer in accordance with the procedure set forth by the manufacturer, Applied Biosystems, Inc., Foster City, California. Restriction enzymes were purchased from New England Biolabs (Beverly, Massachusetts). E. coli DNA polymerase I, Klenow fragment (PolI), T4 DNA Kinase and T4 DNA ligase were purchased from New England Nuclear (Boston, Massachusetts). $^{32}$P-labeled nucleotides were purchased from Amersham (Arlington Heights, Illinois).

E. coli JM101 was obtained from Dr. J. Messing, University of Minnesota (St. Paul, Minnesota) and may be obtained from the American Type Culture Collection (ATCC), Rockville, Maryland under ATCC accession No. 33876. E. coli MM294 may be obtained from the ATCC under ATCC accession No. 33625. E. coli N100 may be obtained from the ATCC under ATCC accession No. 33965. GM48 was obtained from Dr. G. Marinus (University of Massachusetts Medical School, Worchester, Massachusetts).

Restriction enzyme digestions, the T4 DNA ligase reactions, and E. coli DNA polymerase I, Klenow fragment, reactions may be carried out in accordance with the procedures set forth by the manufacturers. Preferred buffers for the following restriction enzymes are as follows. For XbaI, BamHI, EcoRI, PstI, HincII, and HinfI: 50mM NaCl, 6.6mM Tris-HCl, pH 8.0, 6.6mM $MgCl_2$, 5mM dithiothreitol (DTT). For SmaI: 20mM KCl, 6mM Tris-HCl, pH 8.0, 6mM $MgCl_2$, 6mM β-mercaptoethanol. T4 DNA ligase reactions were run in buffers containing 25mM Tris, pH 8.0, 10mM $MgCl_2$, 10mM dithiothritol (DTT), 2mM spermidine and 0.2mM ATP. E. coli DNA polymerase I, Klenow fragment, was used in a buffer containing 20mM Tris, pH 7.2, 10mM $MgCl_2$, 10mM (DTT), 1mM ATP, and 1mM each dATP, dGTP, dCTP, dTTP, (dNTP's). The XbaI linker was obtained from Biolabs (Beverly, Massachusetts).

Alpha-$^{32}$P-dATP (400 Ci/mmol) was added to the Klenow reaction if labeling of the newly synthesized DNA strand was desired.

Oligonucleotides were labeled using gamma-$^{32}$P-ATP (sp. act. greater than 5000 Ci/mmol) and T4 DNA kinase in 100mM Tris, pH 8.0, 10mM $MgCl_2$, 5mM DTT.

Staphylococcus aureus V8 protease was purchased from Miles Scientific (Naperville, Illinois). Factor Xa and thrombin were purchased from Boehringer Mannheim (Indianapolis, Indiana).

Vectors pKO1, pUC18, pBR327, pBR322, M13mp8 and M13mp9 can be purchased from Pharmacia (Piscataway, New Jersey). M13mp18 can be obtained from New England Biolabs (Beverly, Massachusetts). Additionally, pKO1 and pBR322 can be obtained from the ATCC under ATCC accession Nos. 37126 and 37017, respectively. The M13mp9, M13mp8 and pUC18 vectors were obtained from Dr. J. Messing, University of Minnesota (St. Paul, Minnesota). pUC9 and pUC8 can be obtained from Bethesda Research Laboratories, Inc.
(Gaithersburg, Maryland).  Plasmid vector pKO1
described in Chirikjian, J.G. and Papas T. (1981) was
obtained from Dr. Martin Rosenberg (National
Institutes of Health, Bethesda, Maryland).

All bacterial growth media components and
antibiotics were obtained from either Sigma (St.
Louis, Missouri) or Difco Laboratories (Detroit,
Michigan).


                    Example 2

The following example describes the
construction and assembly of the synthetic gene
coding for the 24 amino acids of APIII, shown in
Fig. 1.  The coding portion of the sequence was
preceded by a codon for glutamic acid to provide a
recognition site for cleavage of APIII from a fusion
protein with V8 protease.  The coding portion of the
sequence was preceded by a codon for glumatic acid to
provide a recognition site for cleavage of APIII from
a fusion protein with V8 protease.  The coding
portion of the sequence was immediately followed by
tandem translation termination codons.  Additionally,
recognition sites for several restriction
endonucleases were introduced into the polynucleotide
sequence as shown in Fig. 1.

In order to produce the double-stranded DNA
(dsDNA) fragment shown in Fig. 1, six complementary
and partially overlapping synthetic oligonucleotides
were synthesized as shown in Fig. 3.  Aliquots of the
crude synthetic oligonucleotides were purified by
electrophoresis on polyacrylamide-urea gels, 16%
(w/v) in 7M urea.  See Sanger (1977).  The
concentration of the synthetic DNA in each
preparation was determined by quantitative 5'-end labeling reactions using gamma-$^{32}$P-ATP at a specific activity of 22,000 - 24,000 counts per minute (cpm) per mole of ATP, and T4 DNA Kinase.

The oligonucleotides were then annealed by the following method.  50 picamoles (pmole) of each oligonucleotide was combined in a 25 microliter (μl) final volume reaction mix containing 25mM Tris-HCl, pH 8.0, 10mM MgCl$_2$, 10mM dithiothreitol (DTT), and 0.2mM spermidine, γ-$^{32}$P-ATP at 5000 cpm/pmole of DNA and 40 units of T4 DNA kinase.  The reaction was incubated at 37°C for 30 minutes and then placed in a boiling water bath for two minutes and then allowed to slowly cool to room temperature over a period of six hours.  Four units of T4 DNA ligase and ATP (to 0.4mM) were then added to the products of the annealing reaction and incubated at 4°C for 16 hours to ligate said products.  The ligation reaction was terminated by heating to 70°C for 5 minutes.

The ligation products were then subjected to digestion with EcoRI restriction endonuclease to reduce the maximum size of the DNA fragments to monomers in the event the self-complementary EcoRI ends ligated to form polymers.  The resulting DNA was purified by polyacrylamide gel electrophoresis (PAGE) on a 12% (w/v) gel, 10% (w/v) glycerol and a 93bp molecular weight piece of DNA was electroeluted from the gel to yield the fragment, the complete DNA sequence of which is shown in Fig. 1.

Example 3

The following example describes the construction and expression of two recombinant expression vectors which provide for the production in bacteria of fusion proteins having a V8 protease cleavage site at the junction site or bond.  Specifically, one recombinant expression vector constructed contained a Glu coding sequence following the endogenous protein's DNA (i.e. recA) coding sequence and immediately preceding the coding sequence for the first amino acid (Ser) of the APIII peptide. This fusion construct is hereinafter referred to as recA-Glu-APIII. A second recombinant expression vector constructed contained a DNA sequence coding for Glu-Gly-Arg following the recA DNA coding sequence and immediately preceding the coding sequence for the first amino acid (Ser) of the APIII peptide. This fusion construct is hereinafter referred to as reca-Glu-Gly-Arg-APIII. The entire (100%) recA coding sequence was used in both gene constructs.

The Glu-Gly-Arg junction sequence provides two options for cleavage and subsequent release of a desired peptide, one using blood coagulation factor Xa and the second using V8 protease.

Thus, two distinct gene constructions were made, one coding for a recA-Glu-APIII fusion protein and the second coding for a recA-Glu-Gly-Arg-APIII fusion protein. Expression of the constructs was achieved by transforming an appropriate host cell and culturing the transformants under appropriate conditions as described more fully below.

a.   Creation of a 70% recA-Containing
            Cloning Vector

The recombinant cloning vector pDR1461 comprising a modified pBR322 plasmid carrying the recA promoter and ribosome binding sequences and 70% of the recA DNA coding sequence, collectively referred to as 70% recA, in place of the tetracycline resistance (tet$^r$) gene was constructed as shown in Figure 4.

Specifically, pDR1453 described by Sancar, A. et al. (1980) was isolated from E. coli JM101 transformed with pDR1453 by the methods set forth by Sancar, A. and Rupp, W. D. (1979). E. coli JM101 transformed with pDR1453 has been deposited with the ATCC in accordance with the provisions of the Budapest Treaty and has accordingly been assigned ATCC accession No. 53147.

pDR1453 carries the entire recA gene of E. coli. As shown in Figure 4, 70% recA was excised from pDR1453 as an 1800 bp BamHI/EcoRI fragment and subsequently purified by agarose gel electrophoresis in 0.7% (w/v) agarose, see Maniatis et al. (1981), from which is was then electroeluted. Plasmid pBR322 was cleaved with BamHI and EcoRI and the small BamHI/EcoRI fragment carrying the tet$^r$ gene removed to create a linearized modified pBR322 plasmid. The latter was treated with calf intestine alkaline phosphatase (CAP) and then mixed with the 1800 bp 70% recA containing BamHI/EcoRI fragment in the presence of T4 DNA ligase as shown in Figure 4. The mixture was then incubated overnight at 14°C. Insertion of the 70% recA fragment into the linearized modified pBR322 plasmid was initially ascertained by amp$^r$ colony formation. Insertion of the 70% recA was confirmed by cleavage of isolated recombinant plasmid, Maniatis et al. (1982), with BamHI and EcoRI which yields an 1800 bp fragment comprising the inserted sequence. The 1800 base pair fragment was identified by agarose gel electrophoresis in 0.7% (w/v) agarose as described by Maniatis et al. (1982). All subsequent restriction fragments were identified by this referenced method. The resultant recombinant cloning vector is referred to as pDR1461, shown in Figure 4.

Specifically, pDR1453 described by Sancar, A. et al. (1980) was isolated from E. coli JM101 transformed with pDR1453 by the methods set forth by Sancar, A. and Rupp, W. D. (1979). E. coli JM101 transformed with pDR1453 has been deposited with the ATCC in accordance with the provisions of the Budapest Treaty and has accordingly been assigned ATCC accession No. 53147.

pDR1453 carries the entire recA gene of E. coli. As shown in Figure 4, 70% recA was excised from pDR1453 as an 1800 bp BamHI/EcoRI fragment and subsequently purified by agarose gel electrophoresis in 0.7% (w/v) agarose, see Maniatis et al. (1981), from which is was then electroeluted. Plasmid pBR322 was cleaved with BamHI and EcoRI and the small BamHI/EcoRI fragment carrying the tet$^r$ gene removed to create a linearized modified pBR322 plasmid. The latter was treated with calf intestine alkaline phosphatase (CAP) and then mixed with the 1800 bp 70% recA containing BamHI/EcoRI fragment in the presence of T4 DNA ligase as shown in Figure 4. The mixture was then incubated overnight at 14°C. Insertion of the 70% recA fragment into the linearized modified pBR322 plasmid was initially ascertained by amp$^r$ colony formation. Insertion of the 70% recA was confirmed by cleavage of isolated recombinant plasmid, Maniatis et al. (1982), with BamHI and EcoRI which yields an 1800 bp fragment comprising the inserted sequence. The 1800 base pair fragment was identified by agarose gel electrophoresis in 0.7% (w/v) agarose as described by Maniatis et al. (1982). All subsequent restriction fragments were identified by this referenced method. The resultant recombinant cloning vector is referred to as pDR1461, shown in Figure 4.

### b. Creation of a 70-100% recA-Containing Cloning Vector

Recombinant cloning vector pMON3228 comprising a M13mp9 phage vector carrying the DNA coding sequences for the C-terminal 70% to 100% of recA was constructed as shown in Figures 5 and 6.

pDR1453 was isolated as described above and cleaved with PstI. As shown in Figure 5, an 1800 bp PstI fragment containing a C-terinal portion of the recA coding sequence, as shown in Figure 5, was then isolated and cleaved with HinfI. The sticky HinfI end was then converted to a blunt-end by mixing the digested HinfI fragments with E. coli polymerase I, Klenow fragment, herein referred to as PolI, and deoxynucleotide triphosphates (dNTP's) under appropriate reaction conditions, see e.g. Maniatis et al. (1982), and then cleaved with EcoRI. A 360 bp EcoRI/Hinfi fragment containing the DNA coding sequence for 70-100% recA was then isolated by polyacrylamide gel electrophoresis RF m13mp9 DNA, previously cleaved with EcoRI and SmaI and treated with CAP, was then mixed with the 70-100% recA fragment in the presence of T4 DNA ligase as shown in Figure 5. The ligation mixture was incubated overnight at 14°C. Insertion of the 70-100% recA fragment into M13mp9 was initially ascertained by colorless plaque formation on a lawn of E. coli JM101, grown on 1 x YT medium employing the soft agar overlay procedure described in Maniatis et al. (1982) which included 10 µu 100mM 1PTG (isopropyl-β-D-thiogalactopyranoside) and 50µl 2% (w/v) x-GAL (5-bromo-4-chloro-3-indolyl-β-D-galactopyranoside) in 3 ml of top agar, and transfected with said recombinant vector as described by Messing et al. (1983) and the ssDNA of the recombinant vectors isolated as described by Messing et al. (1982).
Insertion of the 70-100% recA coding sequence was
then confirmed by digestion of recombinant RF DNA
with EcoRI and HindIII which yielded a 380 bp
fragment as shown in Figure 5. The resultant
recombinant vector comprising M13mp9 carrying a DNA
coding sequence for ;70-100% recA was designated
pMON2558.

Next, an EcoRI restriction site was
introduced at the C-terminus of the recA coding
sequence. Briefly, single-stranded DNA of pMON2558
was isolated as described by Messing et al. (1982)
and was employed as a template in the
oligonucleotide-directed site-specific mutagenesis
essentially as described by Zoller and Smith (1982);
Zoller and Smith (1983); Norris et al. (1983).

Figure 6 diagrams the mutagenesis procedure
for creation of an EcoRI restriction site at the
C-terminus of the recA coding sequence.
Specifically, the codon for recA amino acid residue
number 352 was changed from one coding for aspartic
acid (GAT) to one coding for phenylalanine (TTC).
The mutagenesis was conducted as follows. An
oligonucleotide primer shown in Figure 6 containing
the sequence of the desired mutation was used to
prime synthesis of a closed-circular DNA copy of the
ssDNA pMON2558 template. The closed-circular dsDNA
molecules thus generated are separated from incomplete
and ssDNA circles by alkaline sucrose
gradient centrifugation as described by Zoller and
Smith (1983). The closed-circular dsDNA molecules
were then used to transform E. coli JM101 as
described by Messing et al. (1982) and the resulting
colorless plaques were lifted onto Pall filters
obtained from Pall Ultrafine Filtration Corp. (Glen Cove, New York) and screened for hybridization to a
$^{32}$P-labeled form of the oligonucleotide primer used
to generate the site-specific mutagenesis. The
lifting of said plaques was conducted in accordance
with methods described by the Pall Filter
manufacturer. Hybridization screening was carried
out using nylon Biodyne® filters as described by Pall
Ultrafine Filtration Corp. in their "Protocol Guide
for DNA Transfer to Pall Biodyne™A Nylon Filters"
(1983). Filters were washed at increasing
temperatures until the radiolabeled signal was
removed from a control filter which was prepared with
M13mp9/70-100% recA phage. A typical filter washing
protocol employed a room temperature wash in 6xSSC
(0.9M NaCl and 0.09M NaCitrate) for 10 minutes (min.)
followed by a 50°C wash in 6xSSC for 5 min. and
subsequent washings at temperatures increasing by
5°C. Plaques which hybridized to radiolabeled
oligonucleotide primer at temperatures higher than
the control phage were presumed to carry the newly
created 70-100% recA with C-terminal EcoRI site
coding sequence and were termed potential positives.
Alternatively, individual colorless plaques were
picked from the E. coli JM101 transformations and
grown in 5 milliliters (ml) of 2 x YT medium [1.6%
(w/v) tryptone, 1.0% (w/v) yeast extract, 0.5% (w/v)
NaCl] overnight at 37°C with aeration. Phage DNA,
prepared in accordance with Messing et al. (1982),
was then spotted onto nitrocellulose, hybridized with
radiolabeled primer, and washed in increasing
temperatures as described above. Phage DNA which
showed hybridization temperatures higher than
M13mp9/70-100% recA control plaques were similarly
termed potential positives. Potential positive
plaques from both screening procedures were grown as
described above and used to prepare ss phage DNA, which was then sequenced according to the procedure of Sanger et al. (1977) to confirm that they carried the coding sequence for 70-100% recA with the pMON3228. The frequency of addition of the EcoRI site adjacent the C-terminal amino acid codon for recA was about 2-5%.

### C. Creation of a recA-Glu-APIII Expression Vector

pMON6152, an expression vector capable of producing a recA-Glu-APIII fusion protein in a transformed E. coli host under appropriate conditions was constructed as shown in Figures 7-10. Briefly, a modified pKO1 plasmid vector [pKO1(RI⁻)] was constructed as shown in Figure 7. pKO1(RI⁻) comprises a pKO1 plasmid in which the unique EcoRI restriction site has been removed by digesting pKO1 with EcoRI, converting the sticky-ends to blunt-ends by filling in the ends as previously described, and incubating the blunt-ended linearized plasmid with T4 DNA ligase overnight at 14°C. E. coli JM101 were then transformed with pKO1(RI⁻) as described by Maniatis et al. (1982) and the pKO1(RI⁻) isolated as described in Chirikjian, J. and Papas, T. (1981). The absence of an EcoRI site in pKO1(RI⁻) was confirmed by demonstrating the vector's resistance to EcoRI cleavage.

pKO1(RI⁻) was then cleaved with HindIII and SmaI and the small HindIII/SmaI fragment removed. The linearized pKO1(RI⁻) with its HindIII/SmaI fragment deleted was then treated with CAP. The linearized plasmid contains no BamHI restriction sites. RF Ml3mp9 DNA, isolated as previously described, was cleaved with Hind III and SmaI. The small HindIII/SmaI fragment, herein referred to as the Ml3 linker, carrying an internal BamHI site was

07-21(355)A

isolated by acrylamide gel electrophoresis. The M13 linker was inserted into the linearized pKO1(RI⁻), as shown in Figure 7, by mixing the linker with the linearized plasmid in the presence of T4 DNA ligase and incubating overnight at 14°C. E. coli JM101 were then transformed with the ligation mixture as described by Maniatis et al. (1982).

Insertion of the M13mp9 linker into pKO1(RI⁻) was initially confirmed by growth of amp$^r$ colonies on Luria Bertani (LB) plates containing 200µg/ml ampicillin. Insertion of the linker into the plasmid vector was confirmed by isolating the pKO1(RI⁻)/M13 linker vector as previously described and cleaving with BamHI to demonstrate the introduction of a BamHI site.

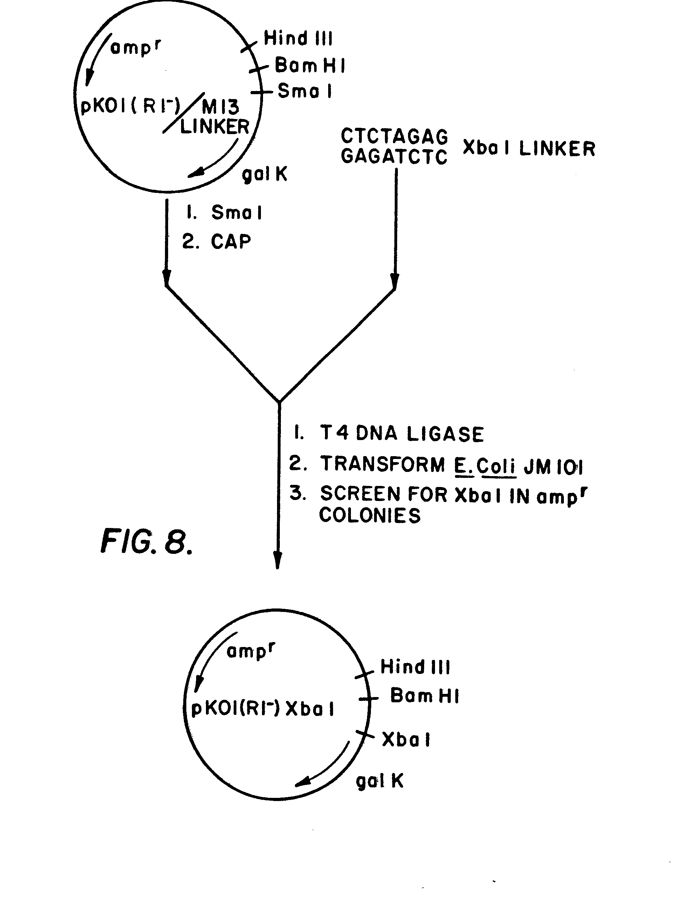

Next, the SmaI site in the pKO1(RI⁻)/M13 linker vector was converted to an XbaI site as shown in Figure 8. Briefly, the pKO1(RI⁻)/M13 linker vector was cleaved with SmaI, treated with CAP, heat inactivated, and then mixed with a synthetic XbaI linker in the presence of T4 DNA ligase as previously described. E. coli JM101 were then transformed with the litigation mixture as previously described. Insertion of the XbaI site was screened for isolating the plasmids from amp$^r$ colonies and demonstrating successful cleavage thereof with XbaI. The resultant plasmid was referred to as pKO1(RI⁻)/XbaI as shown in Figure 8.

Next, the APIII and 70% recA DNA coding sequences were inserted into pKO1(RI⁻)/XbaI at its BamHI site to create recombinant vector pMON6150 as shown in Figure 9. Briefly, pKO1(RI⁻) was cleaved with BamHI and XbaI and the small BamHI/XbaI fragment removed as previously described. The linearized pKO1(RI⁻)/XbaI vector was then mixed, in the presence of T4 DNA ligase, with the 87bp EcoRI/XbaI fragment containing the APIII DNA coding sequence and 1800bp BamHI/EcoRI fragment containing the 70% recA DNA coding sequence which fragments were isolated as shown in Figure 9. Creation of the pMON6150 vector containing the DNA encoding 70% recA fused to DNA encoding APIII was verified by screening plasmids isolated from red amp$^r$ colonies on galactose MacConkey agar plates containing 200μg/ml ampicillin transformed with the ligation mixture for the presence of an 1800 bp BamHI/EcoRI fragment and an 1800 bp BamHI/PvuII fragment as shown in Figure 9.

Expression vector pMON6152, carrying the complete gene for expression of the recA-Glu-APIII fusion protein, was constructed as shown in Figure 10. Briefly, the 280bp EcoRI fragment containing DNA encoding 70-100% recA was isolated from pMON3228 as shown in Figure 10 and inserted into the EcoRI site on pMON6150 as shown in Figure 10. The proper 5' to 3' orientation of the 70-100% recA DNA coding sequence with respect to the APIII and 70% recA DNA coding sequences was confirmed by demonstrating the presence of a 450bp HincII fragment. Improper orientation of the 70-100% recA DNA coding sequence would have resulted in the generation of a 525bp HincII fragment upon cleavage of the newly created recombinant expression vector with HincII.

D.   Construction of the recA-Glu-Gly-Arg-
       APIII Expression Vector

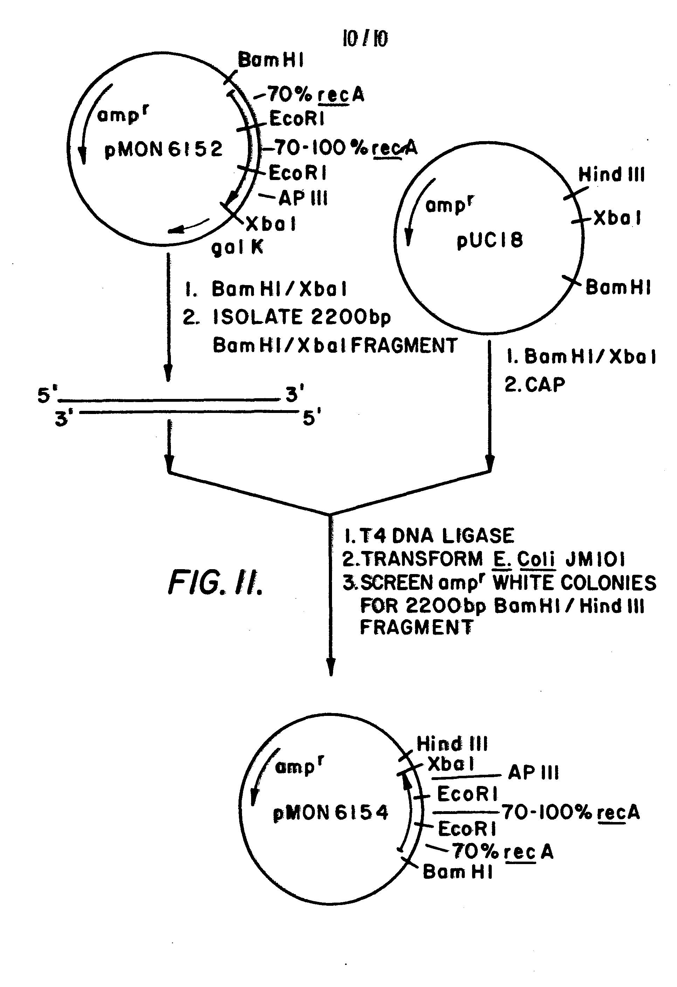

A recA-Glu-Gly-Arg-APIII DNA coding sequence was created by inserting, sequentially, codons for the amino acids glycine (Gly) and arginine (Arg) immediately preceding the N-terminal serine codon of APIII using the techniques of oligonucleotide-directed site-specific mutagenesis previously described. Briefly, pMON6152 transformed into a

0207044

dam⁻host (e.g. a host not expressing a DNA A methylase gene product) was cleaved with BamHI and XbaI and the 2200bp BamHI/XbaI fragment carrying the recA-Glu-APIII fusion gene isolated and inserted into the BamHI to XbaI site in the cloning vector pUC18 as shown in Figure 11 to create pMON6154. The dam⁻host employed was GM48.

The BamHI/HindIII fragment, shown in Figure 11, carrying the recA-Glu-APIII fusion gene was then isolated from pMON6154 and cloned into the BamHI site in M13mp8 and single-stranded recombinant M13mp8 DNA carrying the recA-Glu-APIII gene isolated. An oligonucleotide primer comprising the anti-coding sequence 5'-GAACAGCTGGAACGCCCTTCGAATTCGTT-3' which contained the sequence of the desired mutation (i.e. addition of Gly and Arg codons), immediately preceding the N-terminal serine codon of APIII was then used to prime synthesis of a closed-circular DNA copy of the single-stranded recombinant M13mp8 DNA vector as previously described. Phage DNA carrying the desired Gly-Arg insertion to create a DNA sequence encoding the recA-Glu-Gly-Arg-APIII gene were identified in accordance with the methods previously described. The presence of a DNA sequence encoding a recA-Glu-Gly-Arg-APIII gene was confirmed by DNA sequencing. Positive isolates were then replicated in E. coli JM101, RF recombinant M13Mp8 DNA was then isolated and cleaved with BamHI and HindIII to yield a BamHI/HindIII fragment carrying the recA-Glu-Gly-Arg-APIII gene. The BamHI/HindIII fragment was then inserted into a pBR327 expression vector previously cleaved with BamHI and HindIII to create expression vector pMON6159.

### E.    Expression of recA-Glu-APIII and recA-Glu-Gly-Arg-APIII

E. coli JM101 were then transformed with either pMON6152 or pMON6159 employing the CaCl$_2$ method described by Maniatis et al. (1982) and then induced as follows.  Single colonies of E. coli JM101 carrying either pMON6152 or pMON6159 were inoculated separately and grown overnight at 30°C with aeration. One milliliter (ml) of the overnight cultures was then used to separately inoculate 15ml of M9 media described by Miller, J. H. (1972) supplemented with 1% (w/v) glucose and 0.5% (w/v) casamino acids and grown at 30°C to a density of 150 Klett units.  The cells were then induced by adding nalidixic acid to a final concentration of 50µg/ml and then incubated for 4 hours at 37°C.  Prior to harvesting the cells, an aliquot of 10 Klett mls was then removed from each induced culture and individually lysed in sodium dodecyl sulfate (SDS)-polyacrylamide gel electrophoresis buffer and analyzed by SDS-PAGE in accordance with Laemmli (1970).  Proteins of 41,000 daltons comprising recA-APIII fusion proteins were at high levels in E. coli JM101 from both of the two gene constructs.  E. coli JM101 carrying parental pBR327 and pKO1 plasmids do not produce a protein of 41,000 daltons.  The induced transformed cells were then harvested by pelleting and stored at -70°C as a frozen pellet.

### Example 4

This example demonstrates the cleavage of the Glu-Ser and Glu-Gly-Arg trigger signals by V8 protease.  In this example, the bacterially produced fusion proteins were first isolated from substantially all (about 80-85%) host proteins by immunoaffinity or anion exchange chromatogrphy, subjected to V8 protease cleavage and thereafter the desired peptide, APIII, was purified.

## A. Isolation of recA Fusion Proteins

Both the recA-Glu-APIII and recA-Glu-Gly-Arg-APIII fusion proteins bind to an anion exchange resin such as Fractogel®TSK DEAE-650m at pH 7.0 to 9.0 and are eluted therefrom by 100-300mM NaCl depending upon the pH. The Fractogel®TSK DEAE-650m, hereinafter DEAE TSK, anion exchange resin obtained from E. M. Science (Gibbstown, New Jersey) was prepared as follows. A 500ml DEAE TSK column was prepared and equilibrated with a buffer comprising 100mM NaCl and 50mM Tris-HCl, pH 7.5. A 25 gram quantity of cell paste, isolated as described above, was resuspended in 250mls of 50mM Tris-HCl, pH 7.5, at 8°C. The cell suspension was sonicated using a Heat Systems-Ultrasonic, Inc. (Farmingdale, New York), Model 10-375, containing a 3/4 inch tip, for a total of 6 minutes in 1 minute increments at a maintained temperature ranging from about 8°C to about 18°C. The sonicated suspension was then centrifuged at 26,000xg. for 20 minutes. The supernatant containing about 5 to about 7 grams of protein was decanted from the solid pellet and applied to the surface of the anion exchange column at 8°C at a flow rate of about 5ml per minute. A linear gradient of increasing NaCl concentration ranging from about 100mM NaCl to about 350mM NaCl was applied to the column and fractions containing about 25ml each collected, and was shown to resolve the fusion proteins from about 80% of all E. coli proteins. Elution of proteins from the column was monitored by ultraviolet absorbance at 280mM and by electrophoretic analysis. Those fractions containing fusion protein were combined. The fusion proteins elute at about 280mM NaCl which is substantially higher than that required to elute most E. coli proteins. Alternatively, $NH_4Cl$ was employed to

resolve the fusion proteins from E. coli proteins. A linear gradient of increasing $NH_4Cl$ concentration ranging from about 100mM $NH_4Cl$ to about 350mM $NH_4Cl$ was applied to the column as described for NaCl elution and was shown to resolve the fusion proteins from about 80-85% of all E. coli proteins. The fusion proteins elute at about 200mM to about 230mM $NH_4Cl$.

The recA monoclonal antibody described in a concurrently filed U.S. patent application Serial No. 747,136 by G. G. Krivi and M. L. Bittner entitled "Purification of recA Fusion Peptides" and commonly assigned to Monsanto Company was also successfully applied to yield highly purified recA-Glu-APIII fusion proteins.

### B. V8 protease Cleavage

The fusion protein pool from the DEAE TSK or recA monoclonal antibody columns comprising either the recA-Glu-APIII or recA-Glu-Gly-Arg-APIII fusion protein was dialyzed against 50mM $NH_4HCO_3$. Briefly, approximately 800 to 1000mls of column eluate containing the fusion protein was dialyzed against 24 liters of 50mM $NH_4HCO_3$, pH 7.7 to 8.3, for 6 hours (hrs.) at 4°C and thereafter centrifuged at 12,000 to 15,000xg for 10 min. at 4°C.

Approximately 0.3 to 1.0mg per ml fusion protein was mixed with V8 protease at a molar enzyme to substrate ratio ranging from 1:20 to 1:1000 in 50mM $NH_4HCO_3$, pH 8.0, and incubated at 37°C. A typical cleavage reaction using a 1:500 molar enzyme to substrate ratio was stopped after 4 hours incubation by freezing or by addition of an anion exchange resin which binds the protease as described more fully below.

Approximately 80% of the APIII contained within the recA-Glu-APIII fusion protein and about 50% of the APIII contained within the recA-Glu-Gly-Arg-APIII fusion protein was found to be released as determined by the chick rectum relaxation assay, previously described, and/or by HPLC analysis. Briefly, APIII was resolved from other cleavage reaction peptides on a reverse phase analytical HPLC column containing Vydac™ silica, 18 carbon chain derivatized (5 micron bead size) obtained from the Separations Group (Hesperia, California) using a Waters Associates (Milford, Massachusetts) System. The reverse phase column was equilibrated with 10% (v/v) acetonitrile and 0.5% (v/v) trifluoroacetic acid (TFA) at a flow rate of about 1ml per minute. Following application of the cleavage products to the column, a gradient of increasing acetonitrile ranging from about 10% (v/v) to about 50% (v/v) acetonitrile and 0.05% (v/v) TFA was applied to resolve the peptides. The column was then washed with a gradient of increasing acetonitrile up to 100% (v/v) acetonitrile plus 0.05% (v/v) TFA. APIII eluted from the reverse phase column was quantitated by comparing the area under the peaks to a commercial APIII standard obtained from Peninsula Laboratories (San Carlos, California).

The cleavage reaction mixture was then lyophilized to remove any acetonitrile or $NH_4HCO_3$ and then resolubilized in 10% (v/v) acetonitrile and 0.05% (v/v) TFA prior to APIII purification. Any insoluble species which may have formed during the cleavage reaction were removed by centrifugation at about 12,000 to about 15,000xg for 10 minutes at 4°C.

### C. APIII Purification

The majority of the recA fragments and V8 protease were removed by mixing the cleavage reaction mixture with an anion exchange resin such as DE52 cellulose obtained from Whatman, Limited (Great Britain). The DE52 resin was equilibrated with 50mM $NH_4HCO_3$, pH 8.0, in accordance with manufacturers' instructions at room temperature. Approximately 1 ml of resin was employed for every 2.5 mg of fusion protein and the mixing of resin and cleavage reaction mixture is maintained by stirring for 1 hr. at room temperature. APIII does not bind to the resin and was removed from the resin-containing mixture by filtering the mixture through Whatman No. 1 filter paper obtained from Whatman, Limited (Great Britain) followed by filtration through a 0.45 micron filter unit, such as a Type LS obtained from Nalge Co. (Rochester, New York).

The APIII peptide was further purified on a low pressure reverse phase chromatography system. Briefly, a 50ml column of medium bead size silica such as Vydac™, 15-20 microns, derivatized with 18 carbon chains (C-18) obtained from the Separations Group (Hesperia, California) was employed. The pH of the sample was adjusted to pH 2.5 with TFA and the sample degassed before application to the column. A gradient system of acetonitrile with 0.5% (v/v) TFA, comprising from about 5.0% (v/v) acetonitrile to about 20% (v/v) acetonitrile in 0.2% (v/v) per minute increments was used to resolve the contaminant peptides from APIII. APIII eluted at about 15% (v/v) acetonitrile and the resultant APIII peptide was about 80% free from other proteins or protein fragments. HPLC, as described below, was then employed to further purify the low pressure reverse phase APIII product.

Alternatively, HPLC was employed to purify about 0.5mg APIII directly from the V8 protease cleavage reaction using the Waters Associates (Milford, Massachusetts) reverse phase system described above with the following modifications. A gradient of acetonitrile ranging from about 10% (v/v) to about 40% (v/v) and containing 0.05% (v/v) TFA was applied in 1.0 to 2.0% (v/v) per minute increments at a flow rate of about 2 to 4ml per minute. APIII was found to elute at approximately 30% (v/v) acetonitrile and 0.05% (v/v) TFA.

A single APIII peak was resolved by HPLC from the recA-Glu-APIII V8 protease cleavage reaction; the peak containing 80% of the total APIII obtainable from the fusion protein. Three peaks were resolved by HPLC from the recA-Glu-Gly-Arg-APIII V8 protease cleavage reaction containing approximately 50% of the total APIII obtainable from the fusion protein. Cleavage of both the recA-Glu-APIII and recA-Glu-Gly-Arg-APIII fusion proteins with V8 protease resulted in the endogenous protein remaining substantially intact such that the released APIII peptide was clearly resolved from any fragments generated in the cleavage reaction.

N-terminal amino acid sequencing of the peak peptides was performed using an Applied Biosystems Protein Sequencer Model 470A (Applied Biosystems, Inc., Foster City, California) in accordance with the method described by Hunkapillar et al. (1983a) and Hunkapillar et al. (1983b).

Table I, below, shows the results of the sequence analysis for both the recA-Glu-APIII and recA-Glu-Gly-Arg-APIII V8 protease digests. The last, C-terminal, 10 amino acids of the recA protein comprising the sequence ...ser-glu-gly-val-ala-glu-thr-asn-glu-phe-COOH, contains three potential (Glu) V8 protease cleavage sites. Additionally, the recA protein contains numerous internal glutamic acid (glu) residues which may be actively cleaved by V8 protease. As shown by the N-terminal sequencing results presented in Table I, below, no evidence for V8 protease cleavage within the 10 C-terminal recA amino acid sequence is seen prior to the release of 80% of the APIII contained within the recA-Glu-APIII fusion protein. Conversely, all three glu residues within the 10 C-terminal amino acids of recA trigger V8 protease cleavage to an almost equivalent extent of V8 protease cleavage at the junction site trigger signal (Glu-Gly-Arg).

These results demonstrate that cleavage of these fusion proteins with V8 protease resulted in a preferred cleavage of the trigger signal at the junction site while the trigger signals within the endogenous protein remained substantially intact thereby providing a clear resolution of the desired heterologous peptide (i.e. APIII). These results further demonstrate a most preferred cleavage of a junction site Glu contained within a recA-Glu-atrial Peptide fusion protein. As shown in Table I, below, fusion proteins having a junction site comprising Glu-Gly-Arg resulted in cleavage at both the junction site glutamic acid bond and at internal recA glutamic acid bonds.

The biological activity of the HPLC purified APIII peptides obtained by V8 protease cleavage of both the recA-Glu-APIII and recA-Glu-Gly-Arg-APIII fusion proteins was confirmed as previously described.

## Table I

N-terminal sequence analysis of recA-Glu-APIII and recA-Glu-Gly-Arg-APIII fusion proteins cleaved with V8 protease.

| Sample | I<br>N-Terminal<br>amino acids | II<br>% of APIII[1] |
| --- | --- | --- |
| recA-Glu-APIII | Ser-Ser | 100% |
| recA-Glu-Gly-Arg-<br>APIII | Gly-Val<br>Thr-Asn<br>Phe-Glu<br>Gly-Arg | 5<br>15<br>40<br>40 |

[1] The amount of peptide with the N-terminal sequence shown in column I is shown as a percent (%) of the total APIII in the sample.

## Example 5

This example demonstrates the cleavage at a Phe-Glu-Gly-Arg trigger signal with factor Xa. This trigger signal represents a novel factor Xa recognition site effective for the site-specific release of atrial peptides from recA-atrial peptide fusion proteins. In this example, the fusion protein was first isolated by anion exchange chromatography, subjected to factor Xa cleavage and then the desired peptide, APIII, was purified.

### A.   Isolation of RecA Fusion

The recA-Glu-Gly-Arg-APIII fusion was purified by anion exchange chromatography as described in Example 4.  The fusion protein eluted at about 200mM to about 230mM NH$_4$Cl.

### B.   Factor Xa Cleavage

The fusion protein pool at a protein concentration of approximately 0.3mg/ml was mixed with factor Xa (Boehringer Mannheim; Indianapolis, Indiana) at a molar enzyme to substrate ratio ranging from about 1:20 to about 1:100 in buffer comprising 50mM Tris-HCl, pH 7.5, and 200-230mM NH$_4$Cl.  Calcium chloride at a final concentration of 5mM-50mM and approximately 20μM (final concentration) phospholipid vesicles were added to enhance the rate of cleavage. The vesicles were prepared with Folch fraction III (Sigma Chemical; St. Louis, Missouri) and phosphatidyl inositol at a ratio of 90:1.  The crude lipids were extracted with acetone then a 2:1 molar ratio of chloroform:methanol was used to solubilize the phospholipid and the resultant solution was dried under a stream of nitrogen gas.  The phospholipid was washed twice with ether to remove residual chloroform.  Buffer comprising 50mM Tris-Cl, pH 8.0, was added to the phospholipid to yield a final 25mM phosphate concentration.  The solution was then sonicated in an ice bath until clear.  The cleavage reaction was incubated at 25°C for 4-20 hours and stopped by freezing or by immediately purifying the product peptide.

### C.   APIII Purification

The recA fragment and factor Xa were removed and the APIII purified to homogeneity by reverse phase HPLC as described above.

Approximately 80% of the APIII contained within the fusion was released as determined by chick rectum relaxation activity and analytical reverse phase HPLC peak areas as described in Example 4. Few if any other peptide-like fragments other than APIII were specifically released from the fusion with no other major cleavage occurring in the fusion.

The biological activity of the HPLC purified APIII peptides obtained by factor Xa cleavage of the fusion proteins was confirmed as previously described.

## Example 6

This example demonstrates the expression and cleavage of fusion proteins containing a Glu-Ser trigger signal present at the junction site between either 100% recA and API or bacteria 100% recA and APIV.

## A. Creation of Expression Vectors

A recA-Glu-API DNA coding sequence was created by using the technique of oligonucleotide-directed site-specific mutagenesis as previously described. Specifically, the codons for amino acids 22 through 24 (Phe, Arg, Tyr) were removed from the Glu-APIII gene, to create Glu-API. To accomplish this, the M13mp9/APIII ssDNA, shown in Figure 2, was employed as a template for oligonucleotide-directed site-specific mutagenesis. An oligonucleotide primer comprising the coding sequence 5'-TTGGGTGTAACTCTTTAA-TGATCTAGAGA-3' which contained the sequence of the desired mutation (e.g. deletion of the Phe, Arg, Tyr codons, immediately preceding the termination codons) was then used to prime synthesis of a closed-circular DNA copy of the single-stranded recombinant Ml3mp9 DNA. The presence of a DNA sequence encoding the Glu-API gene was confirmed by DNA sequencing. Positive mutant phage were then replicated in E. coli JM 101 strain and double-stranded replicative form (RF) Ml3mp9 mutant DNA was then isolated and cleaved with EcoRI to yield an EcoRI fragment carrying the Glu-API gene. The EcoRI fragment was then inserted into pMON6075, a pUC9 expression vector, previously cleaved with EcoRI and carrying the recA gene on a BamHI/EcoRI fragment, obtained from pMON6152. Clones isolated from this transformation were screened by cleaving with the restriction enzymes EcoRI and ClaI independently to determine the presence and orienta_ tion of the API gene. A positive clone created the expression vector pMON6163 comprising recA-Glu-API.

A recA-Glu-APIV DNA coding sequence was created by inserting a synthetic DNA fragment into the EcoRI/PvuII restriction sites of the APIII gene (See Figure I). The synthetic DNA fragment contained the codons for the amino acids Glu-Ser-Leu-Arg-Arg to be inserted immediately preceding the N-terminal serine codon of APIII. The coding sequence of the synthetic DNA was as follows:

　　　　　5'- AA TTC GAA TCC CTG CGC CGT TCC AG -3'
　　　　　　　Glu-Phe-Glu-Ser-Leu-Arg-Arg-Ser-Ser The expression vector pMON6159 was cleaved with EcoRI and PvuII and the large linear fragment ligated to the synthetic DNA fragment and transformed into E. coli JM101 as previously described. The recA-Xa-APIII DNA coding sequence was thereby changed to comprise a recA-Glu-APIV DNA coding sequence. Clones isolated by this procedure were screened by restriction analysis cleaving with EcoRI/HindIII to yield a 124 b.p. fragment. Additionally, the DNA coding sequence was confirmed by DNA sequencing. Specifically, the gene of a positive clone was transferred on a BamHI/HindIII fragment to M13mpl8 phage RF DNA that had previously been cleaved with BamHI/HindIII. Single-stranded DNA was prepared from colorless plaque obtained in this transformation and served as the template for dideoxy sequencing (Sanger et. al., 1977). A positive clone, that was verified by DNA sequencing, created the expression vector comprising pBR327 containing recA-Glu-APIV, and was designated pMON6164.

B. Expression, Purification and Cleavage
        of recA-Glu-API and recA-Glu-APIV

E. coli JM101 were transformed with either pMON6163 or pMON6164 as previously described above. The transformed cells were then induced by adding nalidixic acid to the culture medium as previously described. The high level production (i.e. 10-30% of the total host protein) of fusion proteins comprising recA-Glu-API or recA-Glu-APIV was confirmed as previously described.

Both the 100% recA-Glu-API and 100% recA-Glu-APIV fusion proteins were produced as aggregates contained in inclusion (i.e. refractile) bodies. Examples of methods for purifying such proteins from bacteria are described in U.S. Patents 4,511,502 and 4,511,503. In the present example, one gram of cell paste was resuspended in 20 mls of distilled, deionized water at 8°C. The cell suspension was sonicated using a Heat Systems-Ultrasonic, Inc. (Farmingdale, New York), Model 10-375, containing a 1/2 inch tip, for a total of 3 minutes in 1 minute increments. Temperature was maintained at 8-15°C. The sonicated suspension was then centrifuged at 5000 xg. for 10 minutes. The supernatant was discarded.

The recA-Glu-APIV solid pellet was resuspended in 20 mls 50mM sodium acetate, pH 5.5, at 8°C. This resuspension in 50mM sodium acetate and subsequent pelleting at 7500 x g. was repeated 3 times. The final pellet was then dissolved in 5 mls 9.0M urea, 50mM Tris-HCl, pH 7.5, at 8°C. and then centrifuged at 10,000 xg. to remove any remaining cellular debris. The supernatant was then diluted to a final concentration of 2.0M urea with 50mM Tris-HCl, pH 7.9 and subjected to immunoaffinity chromatography as described below.

A 10 ml recA monoclonal antibody affinity column described in a concurrently filed U.S. Patent Application having U.S. Serial number 747,136 incorporated by reference hereto was equilibrated with 25 mM Tris-HCl, 150 mM NaCl, pH 7.9, at 8°C. The suspension containing the fusion protein was applied to the surface of the column at 8°C at a flow rate of approximately 1 ml per minute. The flow rate was maintained at 1 ml per minute for the entire procedure. Fractions containing about 2 ml were collected. The column eluate was monitored by ultraviolet absorbance at 280 nm and electrophoretic analysis. The column, with the fusion protein bound, was washed with 20-50 ml of 25mM Tris-Cl, 500mM NaCl, pH 7.9, at 8°C. The column was then washed with 25mM Tris-Cl, 150mM NaCl, pH 7.9, at 8°C. The fusion protein was eluted with 200 mM glycine, 150 mM NaCl, pH 2.5, at 8°C. Fractions collected at this point were neutralized with 100 microliters of 1 M Tris.

0207044

The 100% recA-Glu-APIV fusion (0.5ml) after urea solubilization and immunoaffinity chromatography was dialyzed versus three changes of 300ml of 50mM $NH_4HCO_3$ for 20 hours at 4°C. The 100% recA-Glu-API solid pellet was directly solubiled in 7M urea, 50mM Tris-HCl pH 7.5, and then was dialyzed versus two liter changes of 50mM $NH_4HCO_3$, pH8.0 for 20 hours. at 4°C.

After dialysis the fusions were diluted to about 0.5mg/ml protein. The fusions were incubated with a 1 to 500 molar ratio enzyme to substrate of V8 protease. The incubations were for 4 hours at 37°C and the reactions were stopped by freezing.

The fusion cleavage mixtures were analyzed on a Waters Associates HPLC reverse phase system as described previously. The column was equilibrated with 10% (v/v) acetonitrile and 0.05% trifluoroacetic acid (v/v). After applying the sample, a linear gradient of 10% to 50% acetonitrile (v/v) with 0.05% trifluoroacetic acid (v/v) in 1% per minute increments at a flow rate of 1ml per minute was used to separate the API or APIV from the recA fragments. Quantitation was done by comparing the HPLC areas of the API or APIV released from the fusions with commercial API and APIV from Peninsula Laboratories (San Carlos, California). Approximately 80% of the theoretical API and APIV in the fusion was released within 4 hours.

C.  API and APIV Purification

The cleaved fusion mixes were dried and reconstituted with 10% (v/v) acetonitrile with 0.05% (v/v) trifluoroacetic acid. The API and APIV were resolved from contaminating proteins on a semi-preparative 10 x 250mm 5 micron C-18 Vydac column (Separations Group, Hesperia, California). The chromatography conditions were the same as for the analytical chromatography except the flow rates were increased to 2 ml per minute. The peak with API or APIV retention time was collected as it eluted from the column.

Both the API and APIV were bioactive in the chick rectum relaxation assay. Both API and APIV were amino acid sequenced as described above.

## Example 7

This example demonstrates the construction of expression vectors comprising a pBR327 plasmid having inserted therein a gene encoding a fusion protein comprising 100% recA, a thrombin cleavage site and atrial peptide APIII or APIV.

The thrombin cleavage site or trigger signal comprised either the amino acid sequence: $NH_2$-Arg-Ala-Leu-Leu-Ala-Gly-Pro-Arg-COOH or $NH_2$-Gly-Pro-Arg-COOH. The former thrombin trigger signal is hereinafter referred to the "extended thrombin site" and the latter is referred to as the "truncated thrombin site."

An expression vector containing a gene coding for a fusion protein comprising 100% recA, an extended thrombin site and APIV was constructed as follows. A double-stranded (ds) DNA fragment coding for the extended thrombin cleavage site and the first three amino-terminal amino acids of APIV was chemically synthesized in accordance with procedures described above.  The coding strand of the synthetic DNA fragment comprised the sequence:

5'-AA TTC GGT GCT CTC CTG GCT GGC CCG CGT TCC
   Glu-Phe-Arg-Ala-Leu-Leu-Ala-Gly-Pro-Arg-Ser-

CTG CGC CGT TCC AG-3'
Leu-Arg-Arg-Ser-Ser

The synthetic ds DNA fragment was then inserted into pMON6159 previously cleaved with EcoRI and PvuII.  The factor Xa (Glu-Gly-Arg) and APIII coding sequences contained within pMON6159 were thereby converted to DNA sequences coding for an extended thrombin cleavage site and APIV yielding a novel expression vector designated pMON6160.  The creation of pMON6160 was confirmed by restriction endonuclease cleavage analysis and DNA sequencing as previously described.

An expression vector comprising a pBR327 plasmid containing a gene for a fusion protein comprising 100% recA, a truncated thrombin trigger signal and APIII was constructed as follows.  In accordance with procedures previously described, a synthetic DNA fragment was chemically synthesized which fragment contained the following coding sequence:

5'-AA TTC GGG CCG CGT TCC AG-3'
   Glu-Phe-Gly-Pro-Arg-Ser-Ser

The synthetic DNA fragment was then inserted into pMON6159 in place of the EcoRI/PvuII restriction fragment of pMON6159.  The creation of an expression vector, designated pMON6166, containing a gene for a fusion protein comprising 100% recA-Gly-Pro-Arg-APIII was confirmed by restriction endonuclease cleavage and DNA sequencing as previously described.

Example 8

This example demonstrates the expression in bacteria of fusion proteins comprising 100% recA, a thrombin trigger signal and APIII or APIV and the purification of APIII or APIV therefrom.

E. coli JM101 cells were transformed with either pMON6160 or pMON6166 and subsequently induced by addition of nalidixic acid to the culture media, all as previously described.

The fusion proteins were then purified as previously described for 100% recA-Glu-APIV.

Approximately 2 to 5 milliliters (ml) of purified fusion protein was dialyzed in one liter of buffer comprising 50mM Tris-HCl, pH 7.8, 5mM CaCl$_2$ and 0.1M NaCl at 4°C for approximately 20 hours. The fusions were incubated with a 1 to 100 molar (enzyme to substrate) ratio of bovine thrombin obtained from Boehringer Mannheim (Indianapolis, Indiana) at room temperature (about 23°C). The incubation was stopped after about 4 hrs. by freezing the cleavage mixture. The cleavage mixture was analyzed and purified on a C-18 analytical column as previously described for API and APIV fusions. About 25% of the theoretical APIII and 80% of the APIV were released following thrombin cleavage. Both APIII and APIV were bioactive in the chick rectum relaxation assay conducted in accordance with previously described methods. The N-terminal sequence of the APIII and the APIV released from the fusion proteins also confirmed the site-specific release of these peptides from the fusion proteins.

References

1.  Austen and Smith (1976) Biochem. and Biophys.
    Res. Comm. 72:411

2.  Behrens and Brown (1976) Federation Proceedings
    35:Abstract No. 611

3.  Bolivar et al. (1977) Gene 2:95

4.  Chirikjian, J. and Papas, T. eds. (1981) Elsever/
    North Holland, New York, pp. 383-415

5.  Cleveland et al. (1977) J. Biol. Chem 252:1102-
    1106

6.  Craig, R. K. and Hall, L. (1983) Genetic
    Engineering 4 ed. R. Williamson, Academic Press

7.  Currie et al. (1983) Science 221:71-73

8.  deBold et al. (1983) Fed. Proc. 42(3):Abstract
    1870, page 611

9.  Eitner et al. (1982) Mol. Gen. Genet. 185:481.

10. deBold and Flynn (1983) Life Sci. 33:297-302

11. Feinstein, S. et al. (1983) Nucleic Acids
    Research 11:2927-2941

12. Fiers, W. et al. (1976) Nature 260:500

13. Germino, J. and Bastia, D. (1984) Proc. Nat'l.
    Acad. Sci. U.S.A. 81:4692-4696

14. Goeddel et al. (1979) Biochemistry 76:101-110

15. Harris, T.J.R. (1983) in Genetic Engineering
    Vol. 4 (ed. Williamson, R.) p. 127-185, Academic
    London

16. Hausinger and Howard (1982) J. Biol. Chem.
    257:2483-2490

17. Hershfield et al. (1974) Proc. Nat'l. Acad. Sci.,
    U.S.A. 71:3455

18. Houmard and Drapeau (1972a) Proc. Nat'l. Acad.
    Sci. U.S.A. 69:3506-3509

19. Houmard and Drapeau (1972b) J. Biol. Chem.
    247:6720-6726

20. Hunkapiller et al. (1983a) Methods in Enzymology
    91:399-413

21. Hunkapiller et al. (1983b) Methods in Enzymology 91:486-493

22. Ikemura, T. (1982) J. Mol. Biol. 158:573-597

23. Itakura et al. (1977) Science 198:1056-1063

24. Johnson, J.S. (1983) Science 219:632-637

25. Keener, S., McNamee, K. and McEntee, K. (1984) J. Bacteriol. 160:153-160.

26. Laemmli (1970) Nature 227:680-685

27. Loenen et al. (1980) Gene 10:249

28. Magnusson, S. (1971) in The Enzymes ed. Paul D. Boyer, vol. 3, pages 278-321.

29. Magnusson, S. et al.(1975) in Proteases and Biological Control; Reich, E., Rifkin, D.B. and Shaw, E., eds. p.123-149; Cold Spring Harbor Laboratories, New York.

30. Maniatis, Fritsch and Shambrook, eds. (1982) Molecular Cloning: A Laboratory Manual

31. Messing et al. (1982) Gene 19269

32. Messing et al. (1983) Methods in Enzymol. 101:20

33. Miller, J. H. (1972) Exps. in Mol. Gen., Cold spring Harber Publications, p. 431

34. Misono, K. S. et al. (1984) Biochem. Biophys. Res. Comm. 123:444-451

35. Nagai and Thogersen (1984) Nature 309:810-812

36. Norris et al. (1983) Nucleic Acid Research 11:5103-5112

37. Pitts, R. F. (1974) Physiology of the Kidney and Body Fluids, 3rd ed., Chicago, Year Book Medical Publishers, pp. 242-258

38. Rao et al. (1979) Gene 7:79

39. Rosenberg, M. et al. (1968) J. Mol. Biol 31:487-505

40. Rutter, W. J. (1979) in Recombinant DNA and Genetic Experiment eds. Morgan, J. and Whelan, W. J.

0207044

41. Sancar, A. and Rupp, D. (1979) _Proc. Nat'l. Acad. Sci. U.S.A._ 76:3144-3148

42. Sancar et al. (1980) _Proc. Nat'l. Acad. Sci. U.S.A._

43. Sanger et al. (1977) _Proc. Nat'l. Acad. Sci., U.S.A._ 74:5463 - 77:2611-2615

44. Seidah, N. G. et al. (1984) _Proc. Nat'l. Acad. Sci. U.S.A._ 81:2640-2644

45. Shine et al. (1980) _Nature_ 285:456-461

46. Soberon et al. (1978) _Gene_ 4:121

47. Stephien et al. (1983) _Gene_ 24:289-297

48. Sussenfeld, H. M. and Bastia, S. J. (1984) _Biotechnology_ Jan., 76-81

49. Tanaka et al. (1982) _Nucleic Acid Research_ 10:1741-1754

50. Trippodo et al. (1982) _Proc. Soc. Exp. Biol. Med._ 170:502-508

51. White and Samson (1954) _J. Lab. Clin. Med._ 43:475-478

52. Witkin, E. M. (1975) _Bacteriol. Rev._ 4D:869-097

53. Zoller and Smith (1982) _Nucleic Acid Research_ 10:6487-6500

54. Zoller and Smith (1983) _Meth. Enzymol._ 100:468-500

WE CLAIM:

1. A method for producing a heterologous peptide which comprises the steps of expressing in bacteria genomic DNA encoding a fusion protein, said fusion protein comprising a heterologous peptide linked to an endogenous protein at a junction site, wherein both the endogenous protein and the junction site have an endopeptidase cleavage site; recovering said fusion protein; treating said fusion peptide with a suitable endopeptidase such that the endopeptidase cleavage site at said junction site is preferentially cleaved while the endopeptidase cleavage site in the endogenous protein is substantially intact, and obtaining therefrom said desired heterologous peptide.

2. The method of Claim 1 wherein said endogenous protein is a recA protein or fragment thereof.

3. The method of Claim 1 wherein said heterologous peptide is an atrial peptide.

4. The method of Claim 3 wherein said atrial peptide is selected from a group consisting of atrial peptide I, atrial peptide III and atrial peptide IV.

5. The method of Claim 1 wherein said endopeptidase cleavage site comprises a glutamic acid-X peptide bond wherein X is selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine.

6.   The method of Claim 5 wherein X is serine.

7.   The method of Claim 5 or 6 wherein said endopeptidase is V8 protease.

8.   The method of Claim 1 wherein said DNA is contained within a plasmid.

9.   The method of Claim 8 wherein said plasmid is selected from the group consisting of pMON6152, pMON6154, pMON6159 and functional equivalents thereof.

10.   The method of Claim 1 wherein said heterologous peptide contains an amino acid sequence essentially the same as that of a naturally occurring eucaryotic peptide.

11.   The method of Claim 10 wherein said eucaryotic peptide has smooth muscle relaxant activity.

12.   A heterologous peptide produced by the method of Claim 1.

13.   A composition comprising an atrial peptide and containing from about 90% to about 99.5% of said peptide and from about 0.5% to about 10% protein native to bacteria.

14.   A DNA sequence or fragment thereof, wherein the coding strand comprises 5'-TCCAGCTGTTTCG GCGGGAATCGATAGATCGGCGCCCAATCAGGCCTTGGGTGTAACTCTTT CCGTTAC-3'.

15. A method for producing an atrial peptide which comprises the steps of expressing in bacteria genomic DNA encoding a fusion protein, said fusion protein comprising an atrial peptide linked to an endogenous protein at a junction site, wherein both the endogenous protein and the junction site have an endopeptidase cleavage site; recovering said fusion protein; treating said fusion protein with a suitable endopeptidase such that the endopeptidase cleavage site at said junction site is preferentially cleaved while the endopeptidase cleavage site in the endogenous protein is substantially intact; and obtaining therefrom said atrial peptide.

16. The method of Claim 15 wherein said endogenous protein is a recA protein or fragment thereof.

17. The method of Claim 15 wherein said endopeptidase cleavage site comprises a glutamic acid-X peptide bond wherein X is selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine.

18. The method of Claim 17 wherein X is serine.

19. The method of Claim 18 or 19 wherein said endopeptidase is V8 protease.

20. A gene comprising DNA encoding sequentially a promoter that functions in bacteria, a ribosome binding site, a recA protein or fragment thereof, a V8 protease trigger signal, a heterologous peptide and a translation stop signal.

21. The gene of Claim 20 wherein the V8 protease trigger signal comprises a serine codon is immediately preceded by a glutamic acid codon.

22. The gene of Claim 20 wherein said heterologous peptide is an atrial peptide.

23. The gene of Claim 22 wherein said atrial peptide is selected from the group consisting of atrial peptide I, atrial peptide III and atrial IV.

24. A recombinant vector selected from the group consisting of a bacterial plasmid and a bacteriophage having inserted therein the gene of Claim 20.

25. A bacteria having the gene of Claim 20 in its genomic DNA.

26. A heterologous polypeptide comprising a recA protein or portion thereof, a junction site comprising an endopeptidase trigger signal and a peptide having smooth muscle relaxant activity.

27. A heterologous polypeptide of Claim 26 wherein said peptide is an atrial peptide.

28. A heterologous polypeptide of Claim 26 wherein the recA protein comprises 100% recA.

29. A method of Claim 1 wherein said bacteria is a gram negative bacteria.

30. A method of Claim 29 wherein said gram negative bacteria is E. coli.

31. A heterologous polypeptide of Claim 26 wherein the endopeptidase trigger signal is selected from a group consisting of V8 protease, factor Xa and thrombin.

32. A heterologous polypeptide of Claim 31 wherein the V8 protease trigger signal comprises glutamic acid.

33. A heterologous polypeptide of Claim 31 wherein the V8 protease trigger signal comprises Glu-Ser.

34. A heterologous polypeptide of Claim 31 wherein the factor Xa trigger signal comprises Glu-Gly-Arg.

35. A heterologous polypeptide of Claim 31 wherein the thrombin cleavage site comprises Arg-Ala-Leu-Leu-Ala-Gly-Pro-Arg.

36. A heterologous polypeptide of Claim 31 wherein the thrombin cleavage site comprises Gly-Pro-Arg.

37. A heterologous polypeptide of Claim 27 wherein the atrial peptide is selected from a group consisting of API, APIII and APIV.

38. A heterologous polypeptide comprising a recA protein or portion thereof, a junction site comprising a V8 trigger signal and APIII.

39. A heterologous polypeptide of Claim 38 wherein the recA protein comprises about 100% recA.

40. A heterologous polypeptide comprising a recA protein or portion thereof, a junction site comprising a factor Xa trigger signal and APIII.

41. A heterologous polypeptide of Claim 40 wherein the factor Xa trigger signal comprises Glu-Gly-Arg.

42. A heterologous polypeptide comprising a recA protein or portion thereof, a junction site comprising a thrombin cleavage site and an atrial peptide.

43. A heterologous polypeptide of Claim 42 wherein the atrial peptide is selected from a group consisting of APIII and APIV.

44. A heterologous polypeptide of Claim 42 wherein the thrombin trigger signal comprises Arg-Ala-Leu-Leu-Ala-Gly-Pro-Arg.

45. A heterologous polypeptide of Claim 42 wherein the thrombin trigger signal comprises Gly-Pro-Arg.

46. A method for producing atrial peptides in gram negative bacteria comprising:

    a. causing expression of genomic DNA which expression results in the production of fusion protein comprising a <u>rec</u>A protein or portion thereof, a junction site comprising an endonuclease trigger signal and an atrial peptide;

    b. isolating the fusion protein;

    c. cleaving the fusion protein with an endopeptidase which recognizes the endopeptidase trigger signal; and

    d. obtaining therefrom the atrial peptide.

47. A method of Claim 46 wherein the atrial peptide is selected from a group consisting of API, APIII and APIV.

48. A method of Claim 46 wherein the endopeptidase trigger signal is selected from a group consisting of a V8 protease, a factor Xa and a thrombin trigger signal.

49. The method of Claim 46 wherein the V8 protease trigger signal comprises glutamic acid.

50. The method of Claim 46 wherein the V8 protease trigger signal comprises Glu-Ser.

51. The method of Claim 46 wherein the factor Xa trigger signal comprises Glu-Gly-Arg.

52. The method of Claim 46 wherein the thrombin trigger signal comprises Arg-Ala-Leu-Leu-Ala-Gly-Pro-Arg.

53. The method of Claim 46 wherein the thrombin trigger signal comprises Gly-Pro-Arg.

54. The method of Claim 46 wherein the recA protein comprises 100% recA.

55. The method of Claim 46 wherein the gram negative bacteria is E. coli.

56. An expression vector comprising pMON6163.

57. An expression vector comprising pMON6164.

58. An expression vector comprising pMON6160.

59. An expression vector comprising pMON6166.

60. A microorganism having ATCC accession number 53147.

61. An expression vector comprising pMON2790.

```
             1     2     3     4     5     6     7     8     9    10
   glu — phe — glu — ser — ser — cys — phe — gly — gly — arg — ile — asp — arg —
5'— A-A-T-T-C-G-A-A-T-C-C-A-G-C-T-G-T-T-T-C-G-G-C-G-G-G-A-G-A-A-T-C-G-A-T-A-G-A—
     EcoRI                   PvuII                              ClaI
3'—         G-C-T-T-A-G-G-T-C-G-A-C-A-A-A-G-C-C-G-C-C-C-T-C-T-T-A-G-C-T-A-T-C-T


            11    12    13    14    15    16    17    18    19    20
            ile — gly — ala — gln — ser — gly — leu — gly — cys — asn
            A-T-C-G-G-C-G-C-C-C-A-A-T-C-A-G-G-C-C-T-T-G-G-G-T-G-T-A-A-C
                 NarI                         StuI
            T-A-G-C-C-G-C-G-G-G-T-T-A-G-T-C-C-G-G-A-A-C-C-C-A-C-A-T-T-G


            21    22    23    24
            ser — phe — arg — tyr — ter — ter
            T-C-T-T-T-C-C-G-T-T-A-C-T-A-A-T-G-A-T-C-T-A-G-A-G-          —3'
                                              Xbal         EcoRI
            A-G-A-A-A-G-G-C-A-A-T-G-A-T-T-A-C-T-A-G-A-T-C-T-C-T-T-A-A —5'
```

## FIG. 1.

EcoRI

M13mp9
RF DNA

AP III

AATT ⌐══════════⌐TTAA

1. EcoRI
2. CALF INTESTINE
   ALKALINE PHOSPHATASE

3. T4 DNA LIGASE

4. TRANSFORM JM101
5. SELECT FOR COLORLESS PLAQUES
6. SCREEN FOR 93 bp EcoRI FRAGMENT
7. SEQUENCE TO CONFIRM COMPLETE AP III
   INSERTION

EcoRI
Xbal

EcoRI

M13mp9 / AP III

*FIG. 2.*

5'___25___ ∧ ___32___ ∧ ___36___ 3'

3'___25___ ∨ ___32___ ∨ ___36___ 5'

*FIG. 3.*

FIG.4.

*FIG.5.*

FIG.6.

FIG. 7.

FIG. 8.

8 / 10

FIG. 9.

FIG. 10.

FIG. II.

**European Patent Office**

## EUROPEAN SEARCH REPORT

| DOCUMENTS CONSIDERED TO BE RELEVANT | | | EP 86870086.5 |
|---|---|---|---|
| Category | Citation of document with indication. where appropriate, of relevant passages | Relevant to claim | CLASSIFICATION OF THE APPLICATION (Int. Cl.4) |
| A | EP - A2 - 0 089 626 (G.D. SEARLE & CO.)<br><br>* Claim 1 *<br>-- | 1 | C 12 N 15/00<br>C 12 P 21/00<br>C 07 K 15/00<br>C 12 N 1/20 |
| A | EP - A1 - 0 108 045 (MONSANTO COMPANY)<br><br>* Claims 1,3,4,5,9 *<br>-- | 1,2 | (C 12 N 15/00<br>C 12 R 1:19) |
| P,A | EP - A2 - 0 150 126 (IMMUNEX CORPORATION)<br><br>* Abstract *<br>-- | 1 | |
| P,A | EP - A2 - 0 164 273 (SUNTORY LIMITED; MATSUO, HISAYUKI)<br><br>* Claim 21 *<br>---- | 1,3 | |
| | | | **TECHNICAL FIELDS SEARCHED (Int. Cl.4)**<br><br>C 12 N<br>C 12 P<br>C 07 K |

The present search report has been drawn up for all claims

| Place of search | Date of completion of the search | Examiner |
|---|---|---|
| VIENNA | 16-09-1986 | WOLF |